(19) Europäisches Patentamt European Patent Office Office européen des brevets

(11) **EP 4 081 285 B1**

(12) **EUROPEAN PATENT SPECIFICATION**

(45) Date of publication and mention of the grant of the patent:
**08.10.2025  Bulletin 2025/41**

(21) Application number: **20906376.7**

(22) Date of filing: **23.12.2020**

(51) International Patent Classification (IPC):
*A61M 16/16* (2006.01)      *A61M 16/00* (2006.01)
*A61M 16/06* (2006.01)      *A61M 16/08* (2006.01)
*A61M 16/10* (2006.01)      *A61M 16/12* (2006.01)
*A61M 16/20* (2006.01)

(52) Cooperative Patent Classification (CPC):
(C-Sets available)
**A61M 16/16; A61M 16/024; A61M 16/109;**
A61M 16/0051; A61M 16/0066; A61M 16/06;
A61M 16/0816; A61M 16/1055; A61M 16/107;
A61M 16/1085; A61M 16/1095; A61M 16/12;
A61M 16/161; A61M 16/162; A61M 16/208;  (Cont.)

(86) International application number:
**PCT/AU2020/051417**

(87) International publication number:
**WO 2021/127732 (01.07.2021 Gazette 2021/26)**

(54) **APPARATUS FOR HUMIDIFYING A RESPIRATORY GAS**

VORRICHTUNG ZUR BEFEUCHTUNG EINES ATEMGASES

APPAREIL PERMETTANT D'HUMIDIFIER UN GAZ RESPIRATOIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **24.12.2019  AU 2019904930**

(43) Date of publication of application:
**02.11.2022  Bulletin 2022/44**

(73) Proprietor: **ResMed Pty Ltd**
**Bella Vista, New South Wales 2153 (AU)**

(72) Inventors:
• **CORK, Simon Robert**
  **Bella Vista, New South Wales 2153 (AU)**
• **CHAMTIE, Hayat**
  **Bella Vista, New South Wales 2153 (AU)**
• **LUO, Bing**
  **Bella Vista, New South Wales 2153 (AU)**
• **YU, Tzu-Chin**
  **Bella Vista, New South Wales 2153 (AU)**
• **LEAVENS, Benjamin**
  **Bella Vista, New South Wales 2153 (AU)**

(74) Representative: **Vossius & Partner**
**Patentanwälte Rechtsanwälte mbB**
**Siebertstraße 3**
**81675 München (DE)**

(56) References cited:
| | |
|---|---|
| EP-A- 3 045 198 | EP-B1- 1 011 589 |
| WO-A1-2009/156921 | WO-A1-2011/086436 |
| WO-A2-2007/038690 | CN-A- 104 548 301 |
| CN-U- 204 352 325 | US-A1- 2009 194 106 |
| US-A1- 2010 282 247 | US-A1- 2010 300 446 |
| US-A1- 2011 100 363 | US-A1- 2013 174 843 |
| US-A1- 2015 030 317 | US-A1- 2015 054 183 |
| US-A1- 2018 200 469 | US-A1- 2019 143 070 |
| US-A1- 2019 209 802 | US-A1- 2019 209 802 |

(52) Cooperative Patent Classification (CPC): (Cont.)
A61M 2016/0021; A61M 2016/0024;
A61M 2016/0027; A61M 2016/0039;
A61M 2202/0208; A61M 2202/0225;
A61M 2205/0216; A61M 2205/15; A61M 2205/18;
A61M 2205/21; A61M 2205/3317; A61M 2205/3334;
A61M 2205/3365; A61M 2205/3368;
A61M 2205/3379; A61M 2205/3553;
A61M 2205/3561; A61M 2205/3584;

A61M 2205/3592; A61M 2205/3653; A61M 2205/42;
A61M 2205/505; A61M 2205/581; A61M 2205/582;
A61M 2205/583; A61M 2205/7518;
A61M 2205/8206; A61M 2206/14; A61M 2209/086

C-Sets
A61M 2202/0208, A61M 2202/0007;
A61M 2202/0225, A61M 2202/0085

**Description**

2 BACKGROUND OF THE TECHNOLOGY

2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use. The present technology further relates to an apparatus for changing the absolute humidity of a flow of air for delivery to an entrance of the airways of a patient.

2.2 DESCRIPTION OF THE RELATED ART

**2.2.1 Human Respiratory System and its Disorders**

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"Respiratory Physiology"*, by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient $CO_2$ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired $CO_2$ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 2.2.2.3 Supplementary oxygen

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

### 2.2.3 Respiratory therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 $cmH_2O$ relative to ambient pressure. For other forms of therapy, such as the delivery of oxygen, the patient interface may not include a seal sufficient to facilitate delivery to the airways of a supply of gas at a positive pressure of about 10 $cmH_2O$. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Pty Ltd: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Pty Ltd has manufactured the following products that incorporate nasal pillows: SWIFT™ nasal pillows mask, SWIFT™ II nasal pillows mask, SWIFT™ LT nasal pillows mask, SWIFT™ FX nasal pillows mask and MIRAGE LIBERTY™ full-face mask. The following patent applications, assigned to ResMed Pty Ltd, describe examples of nasal pillows masks: International Patent Application WO2004/073,778 (describing amongst other things aspects of the ResMed Pty Ltd SWIFT™ nasal pillows), US Patent Application 2009/0044808 (describing amongst other things aspects of the ResMed Pty Ltd SWIFT™ LT nasal pillows); International Patent Applications WO 2005/063,328 and WO 2006/130,903 (describing amongst other things aspects of the ResMed Pty Ltd MIRAGE LIBERTY™ full-face mask); International Patent Application WO 2009/052,560 (describing amongst other things aspects of the ResMed Pty Ltd SWIFT™ FX nasal pillows).

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 cmH$_2$O).

| RPT Device name | A-weighted sound pressure level dB(A) | Year (approx.) |
|---|---|---|
| C-Series Tango™ | 31.9 | 2007 |
| C-Series Tango™ with Humidifier | 33.1 | 2007 |
| S8 Escape™ II | 30.5 | 2005 |
| S8 Escape™ II with H4i™ Humidifier | 31.1 | 2005 |
| S9 AutoSet™ | 26.5 | 2010 |
| S9 AutoSet™ with H5i Humidifier | 28.6 | 2010 |

One known RPT device used for treating sleep disordered breathing is the S9 Sleep Therapy System, manufactured by ResMed Pty Ltd. Another example of an RPT device is a ventilator. Ventilators such as the ResMed Stellar™ Series of Adult and Paediatric Ventilators may provide support for invasive and non-invasive non-dependent ventilation for a range of patients for treating a number of conditions such as but not limited to NMD, OHS and COPD. Other examples of ventilators include the ResMed Lumis™ Series of non-invasive ventilators and the ResMed Astral™ series of life support ventilators.

The ResMed Elisée™ 150 ventilator and ResMed VS III™ ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

RPT devices may include for example, a high flow therapy device configured to provide a high flow therapy. In this regard, some respiratory therapies may aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired CO$_2$ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air. Humidifiers therefore often have the capacity to heat the flow of air was well as humidifying it.

A range of artificial humidification devices and systems are known; however, they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore, medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

### 2.2.3.5 Oxygen source

Experts in this field have recognized that exercise for respiratory failure patients provides long term benefits that slow the progression of the disease, improve quality of life and extend patient longevity. Most stationary forms of exercise like tread mills and stationary bicycles, however, are too strenuous for these patients. As a result, the need for mobility has long been recognized. Until recently, this mobility has been facilitated by the use of small compressed oxygen tanks or cylinders mounted on a cart with dolly wheels. The disadvantage of these tanks is that they contain a finite amount of oxygen and are heavy, weighing about 50 pounds when mounted.

Oxygen concentrators have been in use for about 50 years to supply oxygen for respiratory therapy. Traditional oxygen concentrators have been bulky and heavy making ordinary ambulatory activities with them difficult and impractical. Recently, companies that manufacture large stationary oxygen concentrators began developing portable oxygen concentrators (POCs). The advantage of POCs is that they can produce a theoretically endless supply of oxygen. In order to make these devices small for mobility, the various systems necessary for the production of oxygen enriched gas are condensed. POCs seek to utilize their produced oxygen as efficiently as possible, in order to minimise weight, size, and power consumption. This may be achieved by delivering the oxygen as series of pulses or "boli", each bolus timed to coincide with the start of inspiration. This therapy mode is known as pulsed or demand (oxygen) delivery (POD), in contrast with traditional continuous flow delivery more suited to stationary oxygen concentrators.

### 2.2.3.6 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.7 Mandibular repositioning

A mandibular repositioning device (MRD) or mandibular advancement device (MAD) is one of the treatment options for sleep apnea and snoring. It is an adjustable oral appliance available from a dentist or other supplier that holds the lower jaw (mandible) in a forward position during sleep. The MRD is a removable device that a patient inserts into their mouth prior to going to sleep and removes following sleep. Thus, the MRD is not designed to be worn all of the time. The MRD may be custom made or produced in a standard form and includes a bite impression portion designed to allow fitting to a patient's teeth. This mechanical protrusion of the lower jaw expands the space behind the tongue, puts tension on the pharyngeal walls to reduce collapse of the airway and diminishes palate vibration.

In certain examples a mandibular advancement device may comprise an upper splint that is intended to engage with or fit over teeth on the upper jaw or maxilla and a lower splint that is intended to engage with or fit over teeth on the upper jaw or mandible. The upper and lower splints are connected together laterally via a pair of connecting rods. The pair of connecting rods are fixed symmetrically on the upper splint and on the lower splint.

In such a design the length of the connecting rods is selected such that when the MRD is placed in a patient's mouth the mandible is held in an advanced position. The length of the connecting rods may be adjusted to change the level of protrusion of the mandible. A dentist may determine a level of protrusion for the mandible that will determine the length of the connecting rods.

Some MRDs are structured to push the mandible forward relative to the maxilla while other MADs, such as the ResMed Narval CC™ MRD are designed to retain the mandible in a forward position. This device also reduces or minimises dental and temporo-mandibular joint (TMJ) side effects. Thus, it is configured to minimises or prevent any movement of one or more of the teeth.

### 2.2.3.8 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Pty Ltd has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

Table of noise of prior masks (ISO 17510-2:2007, 10 cmH$_2$O pressure at 1m)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage™ (*) | nasal | 29.5 | 21.5 | 1998 |
| ResMed UltraMirage™ | nasal | 36 (3) | 28 (3) | 2000 |
| ResMed Mirage Activa™ | nasal | 32 (3) | 24 (3) | 2002 |
| ResMed Mirage Micro™ | nasal | 30 (3) | 22 (3) | 2008 |
| ResMed Mirage™ SoftGel | nasal | 29 (3) | 22 (3) | 2008 |
| ResMed Mirage™ FX | nasal | 26 (3) | 18 (3) | 2010 |
| ResMed Mirage Swift™ (*) | nasal pillows | 37 | 29 | 2004 |

(continued)

| Mask name | Mask type | A-weighted sound power level dB(A) (uncertainty) | A-weighted sound pressure dB(A) (uncertainty) | Year (approx.) |
|---|---|---|---|---|
| ResMed Mirage Swift™ II | nasal pillows | 28 (3) | 20 (3) | 2005 |
| ResMed Mirage Swift™ LT | nasal pillows | 25 (3) | 17 (3) | 2008 |
| ResMed AirFit P10 | nasal pillows | 21 (3) | 13 (3) | 2014 |
| (* one specimen only, measured using test method specified in ISO 3744 in CPAP mode at 10 cmH$_2$O) | | | | |

Sound pressure values of a variety of objects are listed below

| Object | A-weighted sound pressure dB(A) | Notes |
|---|---|---|
| Vacuum cleaner: Nilfisk Walter Broadly Litter Hog: B+ Grade | 68 | ISO 3744 at 1m distance |
| Conversational speech | 60 | 1m distance |
| Average home | 50 | |
| Quiet library | 40 | |
| Quiet bedroom at night | 30 | |
| Background in TV studio | 20 | |

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular, it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

US 2011/100363 A1 discloses a breathing system that includes a gas source connected to a patient interface via a passageway. An in-line humidifier is disposed along the passageway. The humidifier includes a humidification chamber having a fluid inlet, a fluid outlet, and a reservoir. The humidifier includes a liquid trap comprising a chamber, a fluid inlet in communication with the liquid trap chamber, and a fluid outlet in communication with the liquid trap chamber. The fluid outlet of the liquid trap is in fluid communication with the fluid inlet of the humidification chamber. The liquid trap is constructed and arranged to discourage liquid from back-flowing from the humidifier to the source of gas. For example, a spill height defined by the liquid trap's fluid inlet is elevated relative to a low point of an interior of the liquid trap regardless of an orientation of the humidifier.

US 20191143070 A1 discloses a tub for a humidifier that includes an inner tub configured to hold a supply of water; an outer tub configured to receive the inner tub, the outer tub comprising a bottom and a cavity being formed between the bottom and the inner tub when the inner tub is received in the inner tub; and a valve configured to control a flow of the supply of water from the inner tub to the cavity. The valve is closed to prevent the flow when the inner tub is received in a first position in the outer tub and open to permit the flow when the inner tub is received in a second position in the outer tub.

US 2019/209802 A1 discloses a humidifier that includes a base configured to retain a body of liquid therein, a top cover, and a seal disposed between the top cover and the base. At least a portion of the base is constructed of a heat conducting material. The top cover defines both an inlet and an outlet communicated with an interior of the base. The inlet is configured to receive pressurized breathable gas and the outlet is configured to deliver the pressurized breathable gas with added humidity.

## 3 BRIEF SUMMARY OF THE TECHNOLOGY

The invention is set out in the appended set of claims.
The present technology is directed towards providing medical devices used in the screening, diagnosis, monitoring, amelioration, treatment, or prevention of respiratory disorders having one or more of improved comfort, cost, efficacy, ease of use and manufacturability.
A first aspect of the present technology relates to apparatus used in the screening, diagnosis, monitoring, amelioration, treatment or prevention of a respiratory disorder.
Another aspect of the present technology relates to methods used in the screening, diagnosis, monitoring, amelioration, treatment or prevention of a respiratory disorder.
An aspect of certain forms of the present technology is to provide methods and/or apparatus that improve the compliance of patients with respiratory therapy.
An aspect of one form of the present technology is an apparatus to change the absolute humidity of a flow of air for delivery to an entrance of the airways of a patient, the change being compared to the absolute humidity of ambient air, the apparatus comprising:

a reservoir configured to hold a volume of liquid;

a heating element to create vapour from the liquid;

a chamber to mix the flow of air with the vapour;

a body, the body comprising a first wall structure comprising a chamber inlet port; and

a closure element comprising an air inlet port for pneumatically connecting to a source of the flow of air,

wherein the closure element is secured to the body to provide a sealed gas flow path between the air inlet port and the chamber inlet port, and a liquid trap in the gas flow path.

Another aspect of one form of the present technology is a respiratory treatment apparatus comprising:

a source of a flow of air at positive pressure to ambient;

a body constructed and arranged to be fixed in location in use relative to the source;

an inlet pneumatic connection structure for connecting to the source to receive sealably the flow of air at positive pressure from the source in use;

a container to hold a body of water in use, the container being configured to direct the flow of air so that the flow of air contacts a surface of the body of water in use so that water vapour may transfer from the body of water to the flow of air in use to increase the absolute humidity of the flow of air, the container including a wall constructed at least in part from a material having a relatively high thermal conductivity;

a heating element;

a temperature sensor;

a controller to control the heating element; and

an outlet pneumatic connection structure to receive the flow of air with increased absolute humidity,

wherein the body is configured to hold the container in location close relative to the heating element so that heat energy may transfer from the heating element to the body of water to increase the absolute humidity of the flow of air,

wherein the controller is constructed and arranged to energise the heating element to heat the water without boiling the water,

wherein the respiratory treatment apparatus includes a sealing arrangement so that in use the flow of air with increased absolute humidity received at the outlet pneumatic connection structure has a positive pressure with respect to ambient,

wherein the inlet pneumatic connection structure comprises an air inlet port, wherein the respiratory treatment apparatus comprises a closure element comprising the air inlet port,

wherein the container comprises a container inlet port, and

wherein the closure element is secured to the body to provide a sealed gas flow path between the air inlet port and the container inlet port, and a liquid trap in the gas flow path.

In examples, the body may comprise a chassis and/or housing. In examples, the chassis and/or housing may comprise the chamber. In examples, the chamber and the reservoir may be provided as a single component of the apparatus. In examples, the apparatus may comprise a container to hold a body of water in use (i.e. to function as a reservoir), the container being configured to expose the flow of air to the surface of the body of water in use so that water vapour may transfer from the body of water to the flow of air in use to increase the absolute humidity of the flow of air (i.e. also provide a chamber to mix the flow of air with the vapour). In examples, the chassis and/or housing is configured to hold the container in location. In examples, the container comprises a container inlet port. In examples the chassis and/or housing comprises a first wall structure comprising a chamber inlet port, and the container inlet port is pneumatically connected to the chamber inlet port. In examples, the container inlet port functions as the chamber inlet port.

In examples, the chamber and the reservoir may be provided separately within the apparatus. In examples, the reservoir may feed a supply of water to a separate chamber in which water vapour is produced and mixed with the flow of air. In examples, one or more wicking elements may be provided within or adjacent at least a portion of the air flow path to allow evaporation of water absorbed by the wicking element.

In examples, the first wall structure comprises a chamber inlet port sealing portion surrounding the chamber inlet port and extending towards the closure element. In examples, the chamber inlet port sealing portion comprises a first annular wall.

In examples, the closure element comprises an air inlet port sealing portion surrounding the air inlet port and extending towards the first wall structure. In examples, the chamber inlet port sealing portion comprises a second annular wall.

In examples, at least a portion of the chamber inlet port sealing portion overlaps the air inlet port sealing portion. In examples the chamber inlet port sealing portion extends into the air inlet port sealing portion. In example the air inlet port sealing portion extends into the chamber inlet port sealing portion.

In examples, the chamber inlet port sealing portion seals against the air inlet port sealing portion.

In examples, the liquid trap comprises a superior portion provided above the chamber. In examples the superior portion of the liquid trap is configured such that liquid drains from the superior portion into an inferior portion of the liquid trap. In examples the superior portion of the liquid trap comprises an inclined surface.

In examples, the liquid trap comprises a lateral portion. In examples a height of the lateral portion is less than a greatest height of the liquid trap. In examples the lateral portion of the liquid trap is configured such that liquid drains from the lateral portion into an inferior portion of the liquid trap. In examples the lateral portion extends from the superior portion of the liquid trap.

In examples, the apparatus comprises a resilient seal. In examples the resilient seal comprises a liquid trap portion positioned between the chamber inlet port sealing portion and the air inlet port sealing portion. In examples the liquid trap portion of the resilient seal covers an interior surface of the air inlet port sealing portion facing the liquid trap. In examples the liquid trap portion of the resilient seal is provided on a rim of the air inlet port sealing portion.

In examples, the resilient seal comprises an air inlet port portion provided on the air inlet port. In examples the air inlet portion comprises a lip. In examples the lip projects from the air inlet port portion of the resilient seal towards the first wall structure. In examples the lip projects radially inwardly from the air inlet port portion of the resilient seal.

In examples, the resilient seal comprises a flange portion between the air inlet port portion and the liquid trap portion of the resilient seal. In examples the flange portion covers a surface of the closure element, between the air inlet and the air inlet port sealing portion, and facing the first wall structure.

In examples the liquid trap seal portion of the resilient seal comprises a lip. In examples the lip bears against a surface of the chamber inlet port sealing portion. In examples the lip projects radially inwardly from the liquid trap seal portion of the resilient seal. In examples the lip projects from the liquid trap seal portion of the resilient seal towards the closure element.

In examples, the apparatus comprises a peripheral seal portion configured to seal at least a portion of a periphery of the closure element against the chassis and/or housing. In examples, the resilient seal and the peripheral seal portion are formed as a unitary component.

The first wall structure is generally upright when the apparatus is in an intended working orientation. In examples, the closure element is generally upright when the apparatus is in an intended working orientation.

In examples, when the apparatus is in an intended working orientation, a lowest point of the chamber inlet port is inferior to a lowest point of the air inlet port.

In examples, when the apparatus is in an intended working orientation, a lowest point of the chamber inlet port is superior to an intended water fill level of the chamber.

In examples, a centre of the chamber inlet port is laterally offset from a centre of the air inlet port. In examples, a centre of the chamber inlet port is inferior to a centre of the air inlet port.

In examples, the liquid trap is configured to trap a volume of liquid of between about 5 ml to about 100 ml. In examples, the liquid trap is configured to trap a volume of liquid of between about 10 ml to about 80 ml. In examples, the liquid trap is configured to trap a volume of liquid of between about 30 ml to about 80 ml. In examples, the liquid trap is configured to trap a volume of liquid of about 60 ml.

In examples, the apparatus comprises an inverted receptacle provided to a superior portion of the chamber inlet port when the apparatus is in an intended working orientation. In examples the inverted receptacle extends from the body towards the closure element. In examples the inverted receptacle comprises an open end and a closed end, wherein the open end intersects the chamber inlet port. In examples the open end aligns with a portion of the chamber inlet port. In examples, the inverted receptacle is semicylindrical. In examples the inverted receptacle comprises a drain hole. In examples the drain hole is provided in a superior facing surface of the inverted receptacle when the apparatus is in an intended working orientation.

In examples, the apparatus comprises an interior trap wall extending from the closure element towards the chamber inlet port and surrounding the air inlet port.

In examples, the inverted receptacle extends from the body towards the interior trap wall. In examples the inverted receptacle extends to be proximal the interior trap wall. In examples, a surface of the closed end of the inverted receptacle contacts the interior trap wall.

In examples, the apparatus may comprise a baffle portion positioned between the air inlet port and the chamber inlet port. In examples, the baffle portion is configured to direct liquid flowing from the chamber inlet port in a radial direction. In examples the baffle portion comprises a baffle member facing the chamber inlet port, wherein the baffle member is in a substantially upright orientation when the apparatus is in an intended working orientation.

In examples, the baffle portion may be provided to the closed end of the inverted receptacle. In examples the baffle portion may include a baffle member supported by one or more baffle support members extending from the body towards the closure element.

In examples the baffle portion is shaped to approximate a portion of the air inlet port. In examples the baffle portion is configured to cover a portion of the air inlet port. In examples the baffle portion is configured to cover an inferior portion of the air inlet port when the apparatus is in an intended working orientation.

In examples the apparatus comprises at least one liquid trap partition providing a plurality of liquid trap catchments within the liquid trap.

In examples, the liquid trap comprises a first liquid trap catchment inferior to the chamber inlet port, and a second liquid trap catchment inferior to the first liquid trap catchment when the apparatus is in an inverted from intended working orientation. In examples the at least one liquid trap partition comprises a dividing wall extending above the chamber away from the air inlet port, wherein the first liquid trap catchment is on a first side of the dividing wall, and the second liquid trap catchment is on an opposing side of the dividing wall. In examples an opening is provided between the first liquid trap catchment and the second liquid trap catchment. In examples the opening between the first liquid trap catchment and the second liquid trap catchment is provided at a free end of the dividing wall.

In examples the at least one liquid trap partition comprises an upright intermediary wall and a partition wall extending from the upright intermediary wall towards the body, wherein a superior gap is provided between the chamber inlet port and the partition wall and the superior gap opens into the first liquid trap catchment.

In examples, the at least one liquid trap partition comprises an interior trap wall extending from the closure element towards the chamber inlet port and surrounding the air inlet port to define a third liquid trap catchment around the interior trap wall.

In examples, the apparatus comprises a moveable barrier configured to move between a stored position when the apparatus is in an intended working orientation, and a closed position when the apparatus is in an inverted orientation. In examples, in the closed position the moveable barrier at least impedes flow through one of the chamber inlet port or the air inlet port, and in the stored position the moveable barrier at least impedes flow through the one of the chamber inlet port or the air inlet port to a lesser extent than when in the closed position. In examples the moveable barrier does not impede flow through the one of the chamber inlet port or the air inlet port when in the stored position.

In examples, the moveable barrier is configured to pivot about a pivot, wherein the pivot is positioned inferior to the one of the chamber inlet port of the air inlet port when the apparatus is in an intended working orientation. In examples, the moveable barrier may be configured to slide between the stored position and the closed position.

In examples, the closure element may be configured to interact with a secondary closure element to provide the liquid trap. In examples the secondary closure element may comprise at least the air inlet port for pneumatically connecting to a source of the flow of air. In examples the secondary closure element may be a component of a device configured to provide the source of the flow of air. In examples the secondary closure element may be a component of a Positive Airway Pressure generator configured to be connected to the apparatus. In examples the secondary closure element may be configured to act as an interface between the apparatus and a device configured to provide the source of the flow of air.

Another aspect of one form of the present technology is an apparatus to change the absolute humidity of a flow of air for delivery to an entrance of the airways of a patient, the change being compared to the absolute humidity of ambient air, the apparatus comprising:

a reservoir configured to hold a volume of liquid;

a heating element to create vapour from the liquid;

a chamber to mix the flow of air with the vapour;

an air inlet port for pneumatically connecting to a source of the flow of air;

a chamber inlet port;

a gas flow path between the air inlet port and the chamber inlet port; and

a liquid trap in the gas flow path between the air inlet port and the chamber inlet port,

wherein when the apparatus is in an intended working orientation, a lowest point of the chamber inlet port is inferior to a lowest point of the air inlet port.

Another aspect of one form of the present technology is a respiratory treatment apparatus comprising:

a source of a flow of air at positive pressure to ambient;

a body constructed and arranged to be fixed in location in use relative to the source;

an inlet pneumatic connection structure for connecting to the source to receive sealably the flow of air at positive pressure from the source in use;

a container to hold a body of water in use, the container being configured to direct the flow of air so that the flow of air contacts a surface of the body of water in use so that water vapour may transfer from the body of water to the flow of air in use to increase the absolute humidity of the flow of air, the container including a wall constructed at least in part from a material having a relatively high thermal conductivity;

a heating element;

a temperature sensor;

a controller to control the heating element; and

an outlet pneumatic connection structure to receive the flow of air with increased absolute humidity,

wherein the body is configured to hold the container in location close relative to the heating element so that heat energy may transfer from the heating element to the body of water to increase the absolute humidity of the flow of air,

wherein the controller is constructed and arranged to energise the heating element to heat the water without boiling the water,

wherein the respiratory treatment apparatus includes a sealing arrangement so that in use the flow of air with increased absolute humidity received at the outlet pneumatic connection structure has a positive pressure with respect to ambient,

wherein the inlet pneumatic connection structure comprises an air inlet port,

wherein the container comprises a container inlet port for receiving the flow of air,

wherein the respiratory treatment apparatus comprises a gas flow path between the air inlet port and the container inlet port,

wherein the respiratory treatment apparatus comprises a liquid trap in the gas flow path between the air inlet port and the container inlet port, and

wherein when the apparatus is in an intended working orientation, a lowest point of the container inlet port is inferior to a lowest point of the air inlet port.

An aspect of one form of the present technology is an apparatus to change the absolute humidity of a flow of air for delivery to an entrance of the airways of a patient, the change being compared to the absolute humidity of ambient air, the apparatus comprising:

a reservoir configured to hold a volume of liquid;

a heating element to create vapour from the liquid;

a chamber to mix the flow of air with the vapour;

a body, the body comprising a first wall structure comprising a chamber inlet port; and

a closure element comprising an air inlet port for pneumatically connecting to a source of the flow of air,

wherein the closure element is secured to the body to provide a sealed gas flow path between the air inlet port and the chamber inlet port, and a liquid trap in the gas flow path, and

wherein when the apparatus is in an intended working orientation, a lowest point of the chamber inlet port is inferior to a lowest point of the air inlet port.

Another aspect of one form of the present technology is a device for treating a respiratory disorder comprising:

a Positive Airway Pressure generator; and

an apparatus to change the absolute humidity of a flow of air produced by the Positive Airway Pressure generator for delivery to an entrance of the airways of a patient, wherein the apparatus is configured in accordance with one or more of the aspects of the present technology described herein.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a humidifier tank that may be washed in a home of a patient, e.g., in soapy water, without requiring specialised cleaning equipment.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

## 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

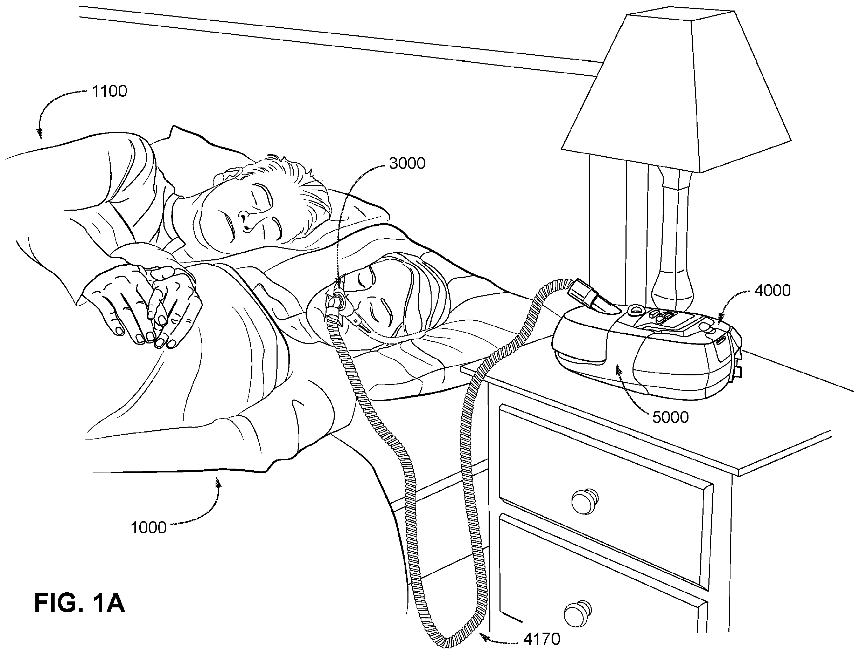

Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.

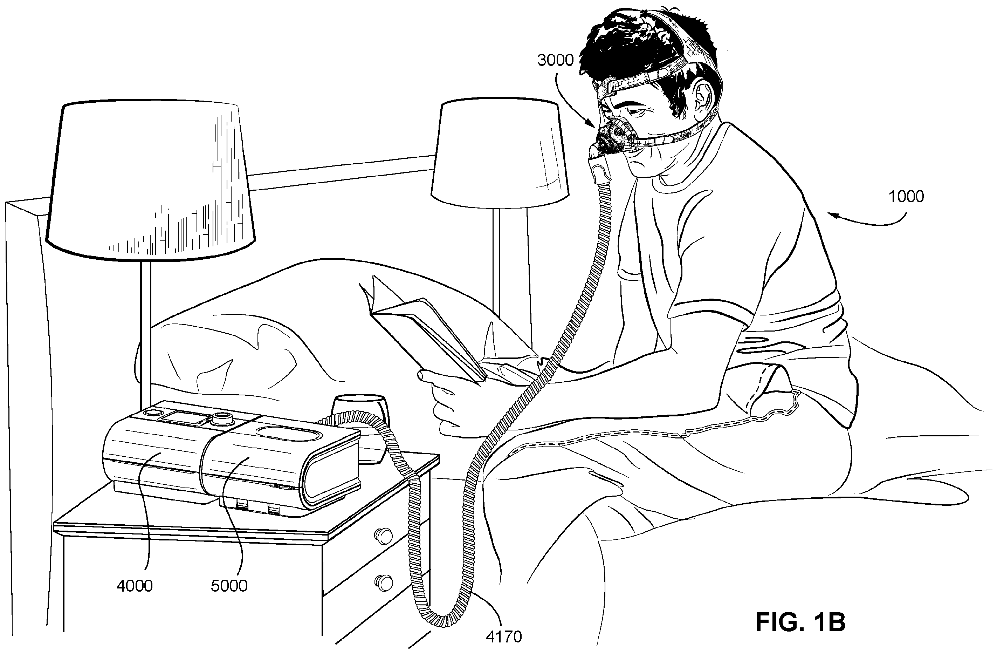

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

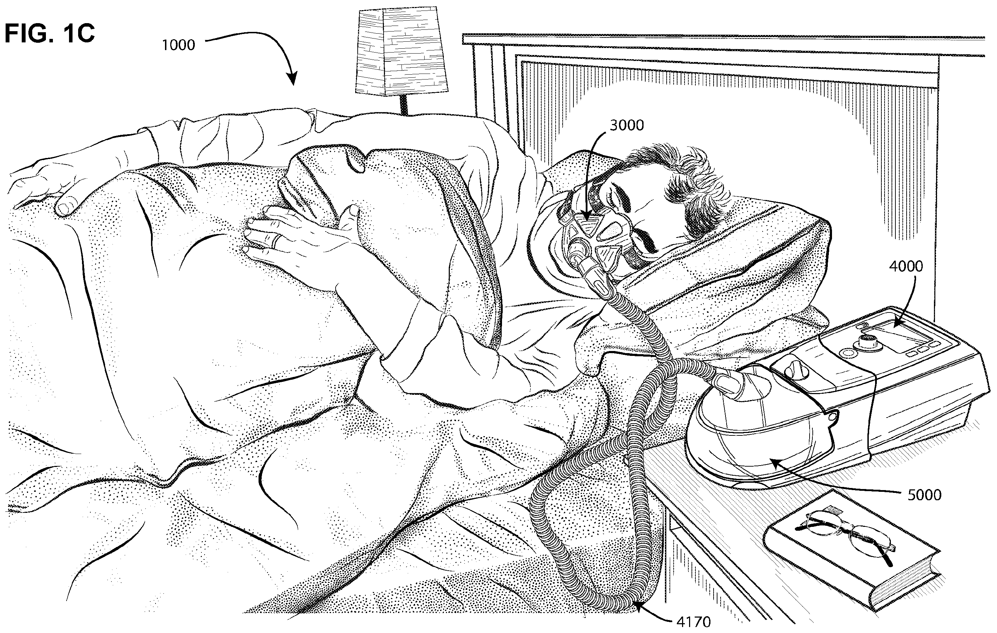

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

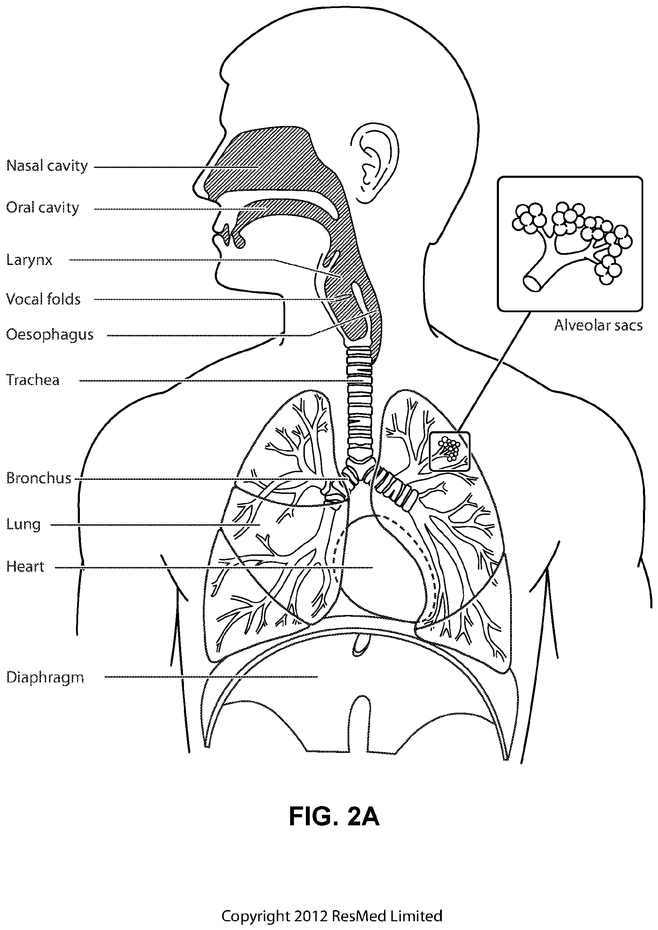

Fig. 2A shows an overview of a human respiratory system including the nasal and oral cavities, the larynx, vocal folds, oesophagus, trachea, bronchus, lung, alveolar sacs, heart and diaphragm.

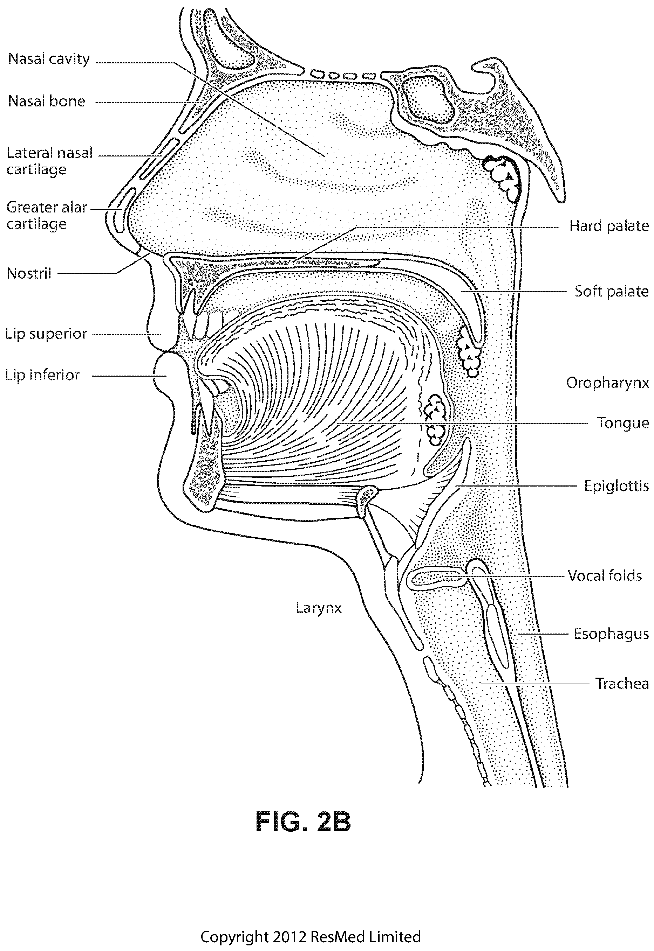

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

### 4.3 PATIENT INTERFACE

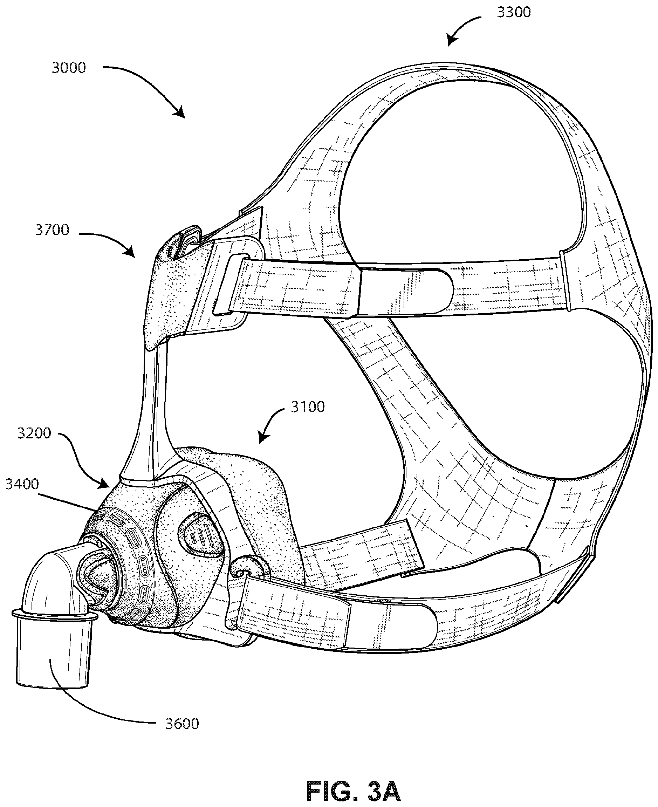

Fig. 3A shows a patient interface in the form of a nasal mask in accordance with one form of the present technology.

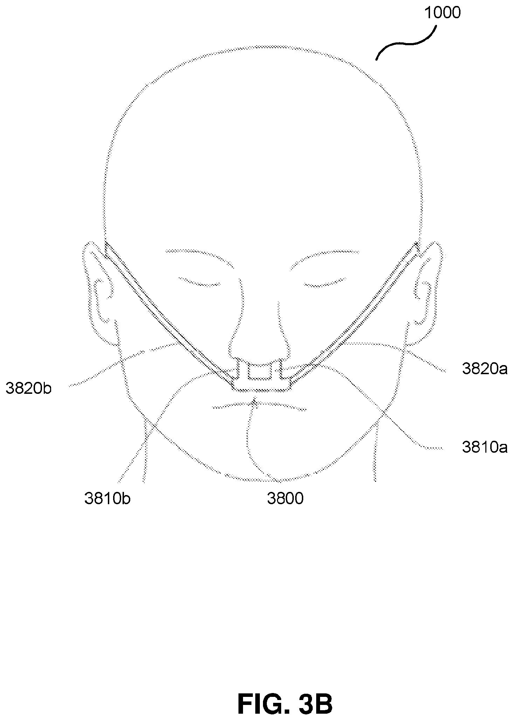

Fig. 3B shows a patient interface in the form of a nasal cannula in accordance with one form of the present technology.

### 4.4 RPT DEVICE

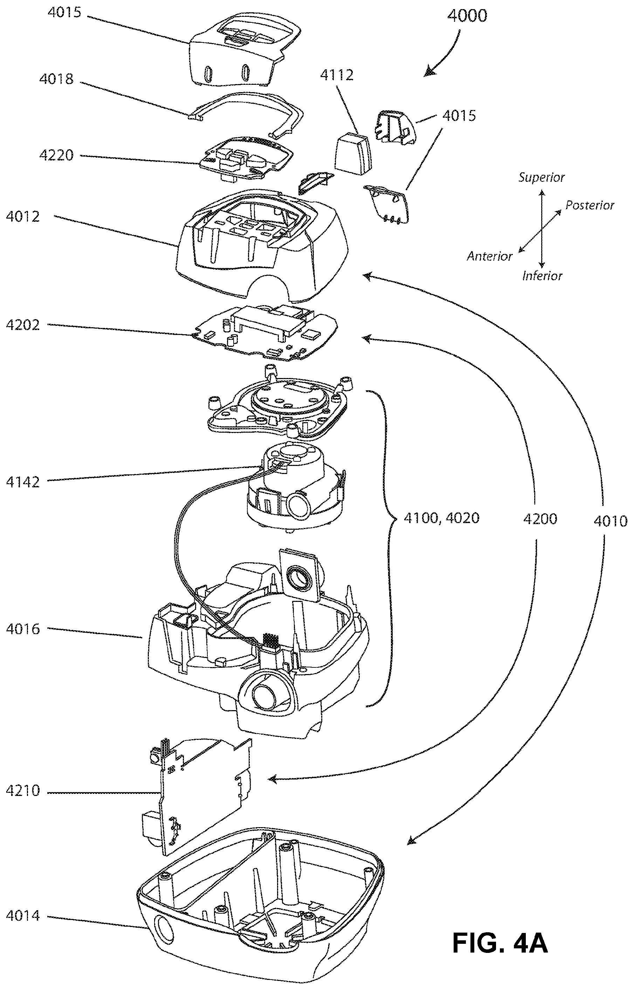

Fig. 4A shows an RPT device in accordance with one form of the present technology.

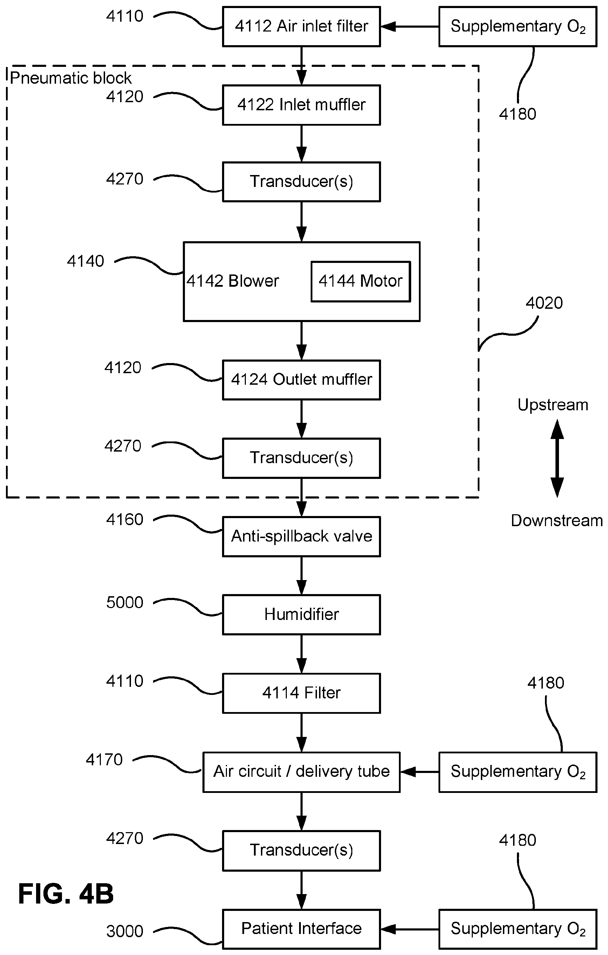

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

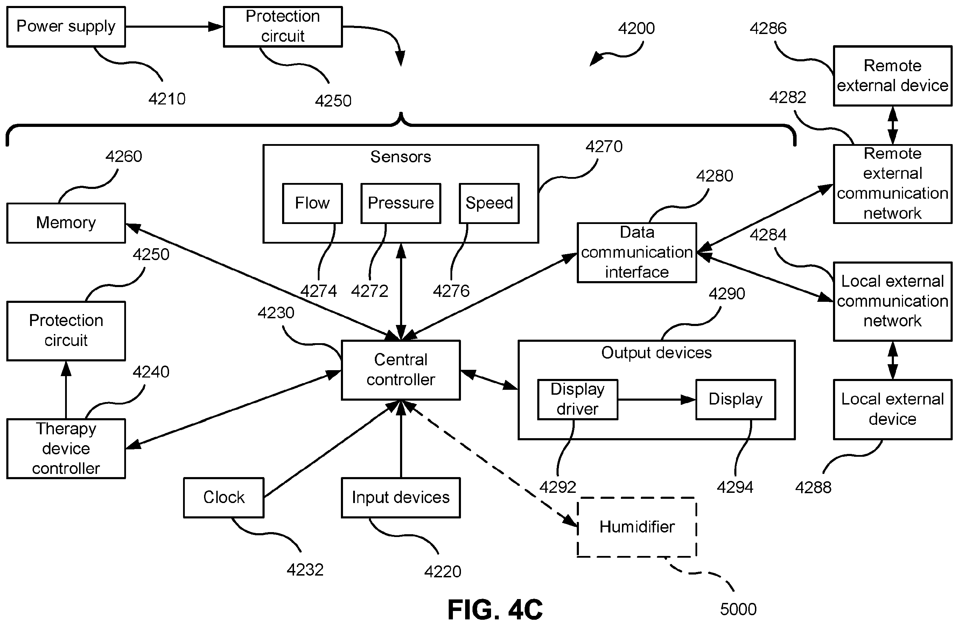

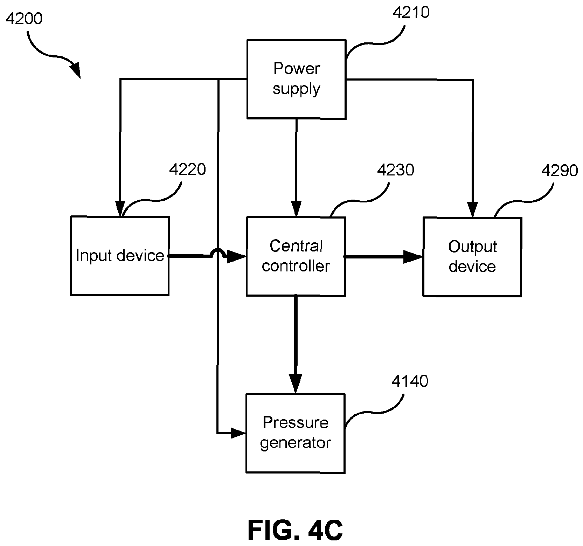

Fig. 4C is a schematic diagram of the electrical components of an RPT device in accordance with one form of the present technology.

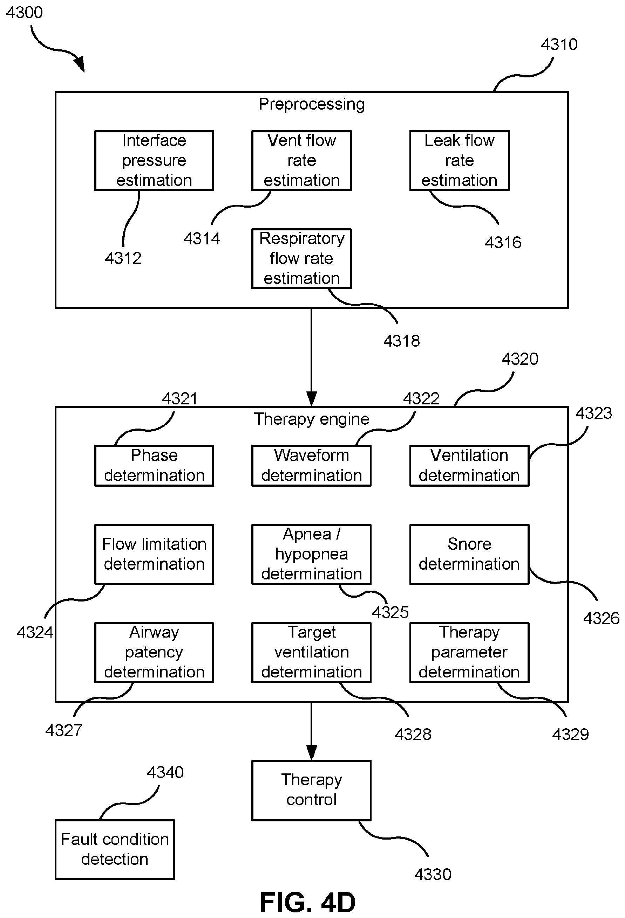

Fig. 4D is a schematic diagram of the algorithms implemented in an RPT device in accordance with one form of the present technology.

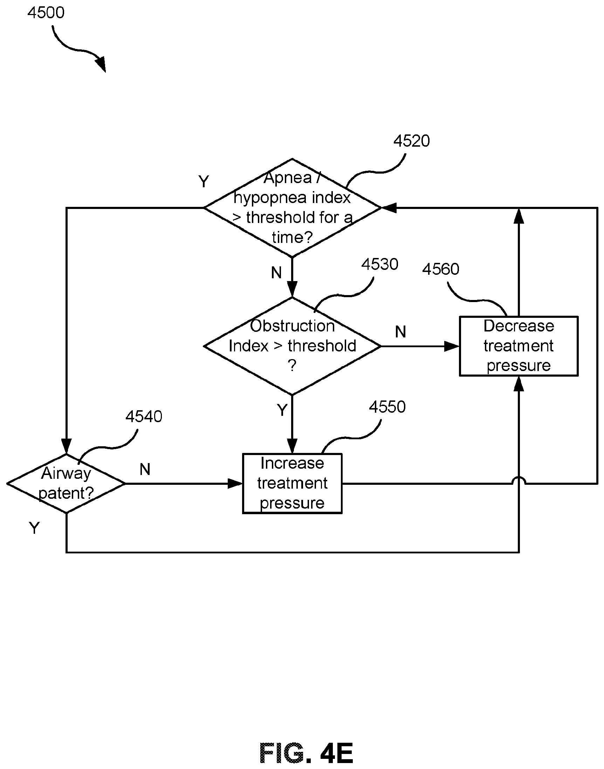

Fig. 4E is a flow chart illustrating a method carried out by the therapy engine module of Fig. 4D in accordance with one form of the present technology.

4.5 HUMIDIFIER

Fig. 5A shows an isometric view of a humidifier in accordance with one form of the present technology.

Fig. 5B shows an isometric view of a humidifier in accordance with one form of the present technology, showing a humidifier reservoir 5110 removed from the humidifier reservoir dock 5130.

Fig. 5C shows a schematic of a humidifier in accordance with one form of the present technology.

Fig. 5D shows an RPT device in accordance with one form of the present technology.

Fig. 5E shows the RPT device of Fig. 5D connected to a releasable component in the form of a humidifier.

Fig. 5F shows the respiratory therapy device and humidifier of Fig. 5E with the lid of the humidifier in an open position.

Fig. 5G shows a cross-section through a housing for a humidifier having a liquid trap in accordance with one form of the present technology.

Fig. 5H shows a schematic of a humidifier having a liquid trap in accordance with one form of the present technology.

Fig. 5I shows an end view of a humidifier having a liquid trap in accordance with one form of the present technology.

Fig. 5J shows a cross-section through a humidifier housing having a liquid trap in in accordance with one form of the present technology, showing the humidifier in a tipped orientation.

Fig. 5K shows a cross-section through a humidifier housing having a liquid trap in in accordance with one form of the present technology, showing the humidifier in a level condition.

Fig. 5L shows a cross-section through a humidifier housing having a liquid trap in in accordance with one form of the present technology.

Fig. 5M shows an end view of an end cap of a humidifier housing in accordance with one form of the present technology.

Fig. 6A shows a perspective view of an inverted receptacle for use in a humidifier having a liquid trap in accordance with one form of the present technology.

Fig. 6B shows a cross-section through a humidifier housing having a liquid trap comprising an inverted receptacle in accordance with one form of the present technology.

Fig. 6C shows a cross-section through a receptacle for use in a humidifier having a liquid trap in accordance with one form of the present technology.

Fig. 6D shows a cross-section through a humidifier housing having a liquid trap comprising an inverted receptacle in accordance with one form of the present technology.

Fig. 7A shows a cross-section through a humidifier housing having a liquid trap in in accordance with one form of the present technology, showing the humidifier in a level orientation.

Fig. 7B shows a cross-section through a humidifier housing having a liquid trap in in accordance with one form of the present technology, showing the humidifier in an upended condition.

Fig. 7C shows a cross-section through a humidifier housing having a liquid trap in in accordance with one form of the present technology.

Fig. 8A shows a perspective view of a receptacle comprising a baffle portion for use in a humidifier having a liquid trap in accordance with one form of the present technology.

Fig. 8B shows a perspective view of the receptacle relative to an air inlet port of a humidifier in accordance with one form of the present technology

Fig. 8C shows a perspective view of a receptacle comprising a baffle portion for use in a humidifier having a liquid trap in accordance with one form of the present technology.

Fig. 8D shows a perspective view of a baffle portion for use in a humidifier having a liquid trap in accordance with one form of the present technology.

Fig. 9 shows a cross-section through a humidifier housing having a liquid trap comprising a liquid trap partition in accordance with one form of the present technology.

Fig. 10 is a front view of a moveable barrier for use in a humidifier having a liquid trap in accordance with one form of the present technology.

Fig. 11 shows a cross-section through a humidifier housing having a liquid trap comprising a secondary closure element in accordance with one form of the present technology.

## 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000 or 3800.

### 5.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

An unsealed patient interface 3800, in the form of a nasal cannula, includes nasal prongs 3810a, 3810b which can deliver air to respective nares of the patient 1000 via respective orifices in their tips. Such nasal prongs do not generally form a seal with the inner or outer skin surface of the nares. The air to the nasal prongs may be delivered by one or more air supply lumens 3820a, 3820b that are coupled with the nasal cannula 3800. The lumens 3820a, 3820b lead from the nasal cannula 3800 to a respiratory therapy device via an air circuit. The unsealed patient interface 3800 is particularly suitable for delivery of flow therapies, in which the RPT device generates the flow of air at controlled flow rates rather than controlled pressures. The "vent" at the unsealed patient interface 3800, through which excess airflow escapes to ambient, is the passage between the end of the prongs 3810a and 3810b of the cannula 3800 via the patient's nares to atmosphere.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 $cmH_2O$ with respect to ambient.

The patient interface 3000 in accordance with one form of the present technology is constructed and arranged to be able to provide a supply of air at a positive pressure of at least 10 $cmH_2O$ with respect to ambient.

The patient interface 3000 in accordance with one form of the present technology is constructed and arranged to be able to provide a supply of air at a positive pressure of at least 20 $cmH_2O$ with respect to ambient.

5.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 $cmH_2O$, or at least 10$cmH_2O$, or at least 20 $cmH_2O$.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors 4274.

One or more of the air path items may be located within a removable unitary structure which will be referred to as a pneumatic block 4020. The pneumatic block 4020 may be located within the external housing 4010. In one form a pneumatic block 4020 is supported by, or formed as part of, the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

**5.4.1 RPT device mechanical & pneumatic components**

An RPT device may comprise one or more of the following components in an integral unit. In an alternative form, one or more of the following components may be located as respective separate units.

**5.4.1.1 Air filter(s)**

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000 or 3800.

**5.4.1.2 Muffler(s)**

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000 or 3800.

### 5.4.1.3 Pressure generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH$_2$O to about 20 cmH$_2$O, or in other forms up to about 30 cmH$_2$O when delivering respiratory pressure therapy. The blower may be as described in any one of the following patents or patent applications: U.S. Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller 4240.

In other forms, a pressure generator 4140 may be a piston-driven pump, a pressure regulator connected to a high pressure source (e.g. compressed air reservoir), or a bellows.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000 or 3800.

In one form, a signal from a transducer 4270 may be filtered, such as by low-pass, high-pass or band-pass filtering.

#### 5.4.1.4.1 Flow rate sensor

A flow rate sensor 4274 in accordance with the present technology may be based on a differential pressure transducer, for example, an SDP600 Series differential pressure transducer from SENSIRION.

In one form, a signal generated by the flow rate sensor 4274 and representing a flow rate is received by the central controller 4230.

#### 5.4.1.4.2 Pressure sensor

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal generated by the pressure sensor 4272 is received by the central controller 4230.

#### 5.4.1.4.3 Motor speed transducer

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

### 5.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

### 5.4.2.3 Central controller

In one form of the present technology, the central controller 4230 is one or a plurality of processors suitable to control an RPT device 4000.

Suitable processors may include an x86 INTEL processor, a processor based on ARM® Cortex®-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit.

In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms 4300 expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

### 5.4.2.4 Clock

The RPT device 4000 may include a clock 4232 that is connected to the central controller 4230.

### 5.4.2.5 Therapy device controller

In one form of the present technology, therapy device controller 4240 is a therapy control module 4330 that forms part of the algorithms 4300 executed by the central controller 4230.

In one form of the present technology, therapy device controller 4240 is a dedicated motor control integrated circuit. For example, in one form a MC33035 brushless DC motor controller, manufactured by ONSEMI is used.

### 5.4.2.6 Protection circuits

The one or more protection circuits 4250 in accordance with the present technology may comprise an electrical protection circuit, a temperature and/or pressure safety circuit.

### 5.4.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

### 5.4.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

### 5.4.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

#### 5.4.2.9.1 Display driver

A display driver 4292 receives as an input the characters, symbols, or images intended for display on the display 4294, and converts them to commands that cause the display 4294 to display those characters, symbols, or images.

#### 5.4.2.9.2 Display

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 5.4.3 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

In other forms of the present technology, some portion or all of the algorithms 4300 may be implemented by a controller of an external device such as the local external device 4288 or the remote external device 4286. In such forms, data representing the input signals and / or intermediate algorithm outputs necessary for the portion of the algorithms 4300 to be executed at the external device may be communicated to the external device via the local external communication network 4284 or the remote external communication network 4282. In such forms, the portion of the algorithms 4300 to be executed at the external device may be expressed as computer programs stored in a non-transitory computer readable storage medium accessible to the controller of the external device. Such programs configure the controller of the external device to execute the portion of the algorithms 4300.

In such forms, the therapy parameters generated by the external device via the therapy engine module 4320 (if such forms part of the portion of the algorithms 4300 executed by the external device) may be communicated to the central controller 4230 to be passed to the therapy control module 4330.

### 5.4.3.1 Pre-processing module

A pre-processing module 4310 in accordance with one form of the present technology receives as an input a signal from a transducer 4270, for example a flow rate sensor 4274 or pressure sensor 4272, and performs one or more process steps to calculate one or more output values that will be used as an input to another module, for example a therapy engine module 4320.

In one form of the present technology, the output values include the interface pressure $Pm,$ the respiratory flow rate $Qr,$ and the leak flow rate $Ql.$

In various forms of the present technology, the pre-processing module 4310 comprises one or more of the following algorithms: interface pressure estimation 4312, vent flow rate estimation 4314, leak flow rate estimation 4316, and respiratory flow rate estimation 4318.

#### 5.4.3.1.1 Interface pressure estimation

In one form of the present technology, an interface pressure estimation algorithm 4312 receives as inputs a signal from the pressure sensor 4272 indicative of the pressure in the pneumatic path proximal to an outlet of the pneumatic block (the device pressure $Pd$) and a signal from the flow rate sensor 4274 representative of the flow rate of the airflow leaving the RPT device 4000 (the device flow rate $Qd$). The device flow rate $Qd,$ absent any supplementary gas 4180, may be used as the total flow rate $Qt.$ The interface pressure algorithm 4312 estimates the pressure drop $\Delta P$ through the air circuit 4170. The dependence of the pressure drop $\Delta P$ on the total flow rate $Qt$ may be modelled for the particular air circuit 4170 by a pressure drop characteristic $\Delta P(Q)$. The interface pressure estimation algorithm, 4312 then provides as an output an estimated pressure, $Pm,$ in the patient interface 3000 or 3800. The pressure, $Pm,$ in the patient interface 3000 or 3800 may be estimated as the device pressure $Pd$ minus the air circuit pressure drop $\Delta P.$

#### 5.4.3.1.2 Vent flow rate estimation

In one form of the present technology, a vent flow rate estimation algorithm 4314 receives as an input an estimated pressure, $Pm,$ in the patient interface 3000 or 3800 from the interface pressure estimation algorithm 4312 and estimates a vent flow rate of air, $Qv,$ from a vent 3400 in a patient interface 3000 or 3800. The dependence of the vent flow rate $Qv$ on the interface pressure $Pm$ for the particular vent 3400 in use may be modelled by a vent characteristic $Qv(Pm)$.

#### 5.4.3.1.3 Leak flow rate estimation

In one form of the present technology, a leak flow rate estimation algorithm 4316 receives as an input a total flow rate, $Qt,$ and a vent flow rate $Qv,$ and provides as an output an estimate of the leak flow rate $Ql.$ In one form, the leak flow rate estimation algorithm estimates the leak flow rate $Ql$ by calculating an average of the difference between total flow rate $Qt$ and vent flow rate $Qv$ over a period sufficiently long to include several breathing cycles, e.g. about 10 seconds.

In one form, the leak flow rate estimation algorithm 4316 receives as an input a total flow rate $Qt,$ a vent flow rate $Qv,$ and an estimated pressure, $Pm,$ in the patient interface 3000 or 3800, and provides as an output a leak flow rate $Ql,$ by calculating a leak conductance, and determining a leak flow rate $Ql$ to be a function of leak conductance and pressure, $Pm.$ Leak conductance is calculated as the quotient of low pass filtered non-vent flow rate equal to the difference between total flow rate $Qt$ and vent flow rate $Qv,$ and low pass filtered square root of pressure $Pm,$ where the low pass filter time constant has a value sufficiently long to include several breathing cycles, e.g. about 10 seconds. The leak flow rate $Ql$ may be estimated as the product of leak conductance and a function of pressure, $Pm.$

#### 5.4.3.1.4 Respiratory flow rate estimation

In one form of the present technology, a respiratory flow rate estimation algorithm 4318 receives as an input a total flow rate, $Qt,$ a vent flow rate, $Qv,$ and a leak flow rate, $Ql,$ and estimates a respiratory flow rate of *air, Qr,* to the patient, by subtracting the vent flow rate $Qv$ and the leak flow rate $Ql$ from the total flow rate $Qt.$

**5.4.3.2 Therapy Engine Module**

In one form of the present technology, a therapy engine module 4320 receives as inputs one or more of a pressure, *Pm,* in a patient interface 3000 or 3800, and a respiratory flow rate of air to a patient, *Qr,* and provides as an output one or more therapy parameters.

In one form of the present technology, a therapy parameter is a treatment pressure *Pt.*

In one form of the present technology, therapy parameters are one or more of an amplitude of a pressure variation, a base pressure, and a target ventilation.

In various forms, the therapy engine module 4320 comprises one or more of the following algorithms: phase determination 4321, waveform determination 4322, ventilation determination 4323, inspiratory flow limitation determination 4324, apnea / hypopnea determination 4325, snore determination 4326, airway patency determination 4327, target ventilation determination 4328, and therapy parameter determination 4329.

*5.4.3.2.1 Phase determination*

In one form of the present technology, the RPT device 4000 does not determine phase.

In one form of the present technology, a phase determination algorithm 4321 receives as an input a signal indicative of respiratory flow rate, *Qr,* and provides as an output a phase $\Phi$ of a current breathing cycle of a patient 1000.

In some forms, known as discrete phase determination, the phase output $\Phi$ is a discrete variable. One implementation of discrete phase determination provides a bi-valued phase output $\Phi$ with values of either inhalation or exhalation, for example represented as values of 0 and 0.5 revolutions respectively, upon detecting the start of spontaneous inhalation and exhalation respectively. RPT devices 4000 that "trigger" and "cycle" effectively perform discrete phase determination, since the trigger and cycle points are the instants at which the phase changes from exhalation to inhalation and from inhalation to exhalation, respectively. In one implementation of bi-valued phase determination, the phase output $\Phi$ is determined to have a discrete value of 0 (thereby "triggering" the RPT device 4000) when the respiratory flow rate *Qr* has a value that exceeds a positive threshold, and a discrete value of 0.5 revolutions (thereby "cycling" the RPT device 4000) when a respiratory flow rate *Qr* has a value that is more negative than a negative threshold. The inhalation time *Ti* and the exhalation time *Te* may be estimated as typical values over many respiratory cycles of the time spent with phase $\Phi$ equal to 0 (indicating inspiration) and 0.5 (indicating expiration) respectively.

Another implementation of discrete phase determination provides a tri-valued phase output $\Phi$ with a value of one of inhalation, mid-inspiratory pause, and exhalation.

In other forms, known as continuous phase determination, the phase output $\Phi$ is a continuous variable, for example varying from 0 to 1 revolutions, or 0 to $2\pi$ radians. RPT devices 4000 that perform continuous phase determination may trigger and cycle when the continuous phase reaches 0 and 0.5 revolutions, respectively. In one implementation of continuous phase determination, a continuous value of phase $\Phi$ is determined using a fuzzy logic analysis of the respiratory flow rate *Qr.* A continuous value of phase determined in this implementation is often referred to as "fuzzy phase". In one implementation of a fuzzy phase determination algorithm 4321, the following rules are applied to the respiratory flow rate *Qr:*

1. If the respiratory flow rate is zero and increasing fast then the phase is 0 revolutions.

2. If the respiratory flow rate is large positive and steady then the phase is 0.25 revolutions.

3. If the respiratory flow rate is zero and falling fast, then the phase is 0.5 revolutions.

4. If the respiratory flow rate is large negative and steady then the phase is 0.75 revolutions.

5. If the respiratory flow rate is zero and steady and the 5-second low-pass filtered absolute value of the respiratory flow rate is large then the phase is 0.9 revolutions.

6. If the respiratory flow rate is positive and the phase is expiratory, then the phase is 0 revolutions.

7. If the respiratory flow rate is negative and the phase is inspiratory, then the phase is 0.5 revolutions.

8. If the 5-second low-pass filtered absolute value of the respiratory flow rate is large, the phase is increasing at a steady rate equal to the patient's breathing rate, low-pass filtered with a time constant of 20 seconds.

The output of each rule may be represented as a vector whose phase is the result of the rule and whose magnitude is the fuzzy extent to which the rule is true. The fuzzy extent to which the respiratory flow rate is "large", "steady", etc. is determined with suitable membership functions. The results of the rules, represented as vectors, are then combined by some function such as taking the centroid. In such a combination, the rules may be equally weighted, or differently weighted.

In another implementation of continuous phase determination, the phase Φ is first discretely estimated from the respiratory flow rate $Qr$ as described above, as are the inhalation time $Ti$ and the exhalation time $Te$. The continuous phase Φ at any instant may be determined as the half the proportion of the inhalation time $Ti$ that has elapsed since the previous trigger instant, or 0.5 revolutions plus half the proportion of the exhalation time $Te$ that has elapsed since the previous cycle instant (whichever instant was more recent).

### 5.4.3.2.2 Waveform determination

In one form of the present technology, the therapy parameter determination algorithm 4329 provides an approximately constant treatment pressure throughout a respiratory cycle of a patient.

In other forms of the present technology, the therapy control module 4330 controls the pressure generator 4140 to provide a treatment pressure $Pt$ that varies as a function of phase Φ of a respiratory cycle of a patient according to a waveform template $\Pi(\Phi)$.

In one form of the present technology, a waveform determination algorithm 4322 provides a waveform template $\Pi(\Phi)$ with values in the range [0, 1] on the domain of phase values Φ provided by the phase determination algorithm 4321 to be used by the therapy parameter determination algorithm 4329.

In one form, suitable for either discrete or continuously-valued phase, the waveform template $\Pi(\Phi)$ is a square-wave template, having a value of 1 for values of phase up to and including 0.5 revolutions, and a value of 0 for values of phase above 0.5 revolutions. In one form, suitable for continuously-valued phase, the waveform template $\Pi(\Phi)$ comprises two smoothly curved portions, namely a smoothly curved (e.g. raised cosine) rise from 0 to 1 for values of phase up to 0.5 revolutions, and a smoothly curved (e.g. exponential) decay from 1 to 0 for values of phase above 0.5 revolutions. In one form, suitable for continuously-valued phase, the waveform template $\Pi(\Phi)$ is based on a square wave, but with a smooth rise from 0 to 1 for values of phase up to a "rise time" that is less than 0.5 revolutions, and a smooth fall from 1 to 0 for values of phase within a "fall time" after 0.5 revolutions, with a "fall time" that is less than 0.5 revolutions.

In some forms of the present technology, the waveform determination algorithm 4322 selects a waveform template $\Pi(\Phi)$ from a library of waveform templates, dependent on a setting of the RPT device. Each waveform template $\Pi(\Phi)$ in the library may be provided as a lookup table of values $\Pi$ against phase values Φ. In other forms, the waveform determination algorithm 4322 computes a waveform template $\Pi(\Phi)$ "on the fly" using a predetermined functional form, possibly parametrised by one or more parameters (e.g. time constant of an exponentially curved portion). The parameters of the functional form may be predetermined or dependent on a current state of the patient 1000.

In some forms of the present technology, suitable for discrete bi-valued phase of either inhalation (Φ = 0 revolutions) or exhalation (Φ = 0.5 revolutions), the waveform determination algorithm 4322 computes a waveform template Π "on the fly" as a function of both discrete phase Φ and time t measured since the most recent trigger instant. In one such form, the waveform determination algorithm 4322 computes the waveform template $\Pi(\Phi, t)$ in two portions (inspiratory and expiratory) as follows:

$$\Pi(\Phi, t) = \begin{cases} \Pi_i(t), & \Phi = 0 \\ \Pi_e(t - T_i), & \Phi = 0.5 \end{cases}$$

where $\Pi_i(t)$ and $\Pi_e(t)$ are inspiratory and expiratory portions of the waveform template $\Pi(\Phi, t)$. In one such form, the inspiratory portion $\Pi_i(t)$ of the waveform template is a smooth rise from 0 to 1 parametrised by a rise time, and the expiratory portion $\Pi_e(t)$ of the waveform template is a smooth fall from 1 to 0 parametrised by a fall time.

### 5.4.3.2.3 Ventilation determination

In one form of the present technology, a ventilation determination algorithm 4323 receives an input a respiratory flow rate $Qr$, and determines a measure indicative of current patient ventilation, $Vent$.

In some implementations, the ventilation determination algorithm 4323 determines a measure of ventilation $Vent$ that is an estimate of actual patient ventilation. One such implementation is to take half the absolute value of respiratory flow rate, $Qr$, optionally filtered by low-pass filter such as a second order Bessel low-pass filter with a corner frequency of 0.11 Hz.

In other implementations, the ventilation determination algorithm 4323 determines a measure of ventilation $Vent$

that is broadly proportional to actual patient ventilation. One such implementation estimates peak respiratory flow rate *Qpeak* over the inspiratory portion of the cycle. This and many other procedures involving sampling the respiratory flow rate *Qr* produce measures which are broadly proportional to ventilation, provided the flow rate waveform shape does not vary very much (here, the shape of two breaths is taken to be similar when the flow rate waveforms of the breaths normalised in time and amplitude are similar). Some simple examples include the median positive respiratory flow rate, the median of the absolute value of respiratory flow rate, and the standard deviation of flow rate. Arbitrary linear combinations of arbitrary order statistics of the absolute value of respiratory flow rate using positive coefficients, and even some using both positive and negative coefficients, are approximately proportional to ventilation. Another example is the mean of the respiratory flow rate in the middle K proportion (by time) of the inspiratory portion, where $0 < K < 1$. There is an arbitrarily large number of measures that are exactly proportional to ventilation if the flow rate shape is constant.

### 5.4.3.2.4 Determination of Inspiratory Flow limitation

In one form of the present technology, the central controller 4230 executes an inspiratory flow limitation determination algorithm 4324 for the determination of the extent of inspiratory flow limitation.

In one form, the inspiratory flow limitation determination algorithm 4324 receives as an input a respiratory flow rate signal *Qr* and provides as an output a metric of the extent to which the inspiratory portion of the breath exhibits inspiratory flow limitation.

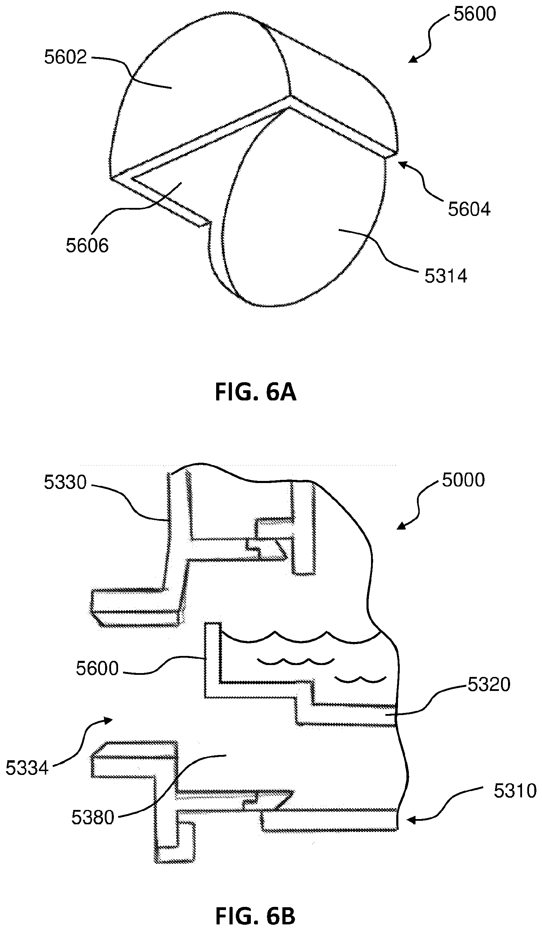

In one form of the present technology, the inspiratory portion of each breath is identified by a zero-crossing detector. A number of evenly spaced points (for example, sixty-five), representing points in time, are interpolated by an interpolator along the inspiratory flow rate-time curve for each breath. The curve described by the points is then scaled by a scalar to have unity length (duration/period) and unity area to remove the effects of changing breathing rate and depth. The scaled breaths are then compared in a comparator with a pre-stored template representing a normal unobstructed breath, similar to the inspiratory portion of the breath shown in Fig. 6A. Breaths deviating by more than a specified threshold (typically 1 scaled unit) at any time during the inspiration from this template, such as those due to coughs, sighs, swallows and hiccups, as determined by a test element, are rejected. For non-rejected data, a moving average of the first such scaled point is calculated by the central controller 4230 for the preceding several inspiratory events. This is repeated over the same inspiratory events for the second such point, and so on. Thus, for example, sixty five scaled data points are generated by the central controller 4230, and represent a moving average of the preceding several inspiratory events, e.g., three events. The moving average of continuously updated values of the (e.g., sixty five) points are hereinafter called the "scaled flow rate ", designated as *Qs(t)*. Alternatively, a single inspiratory event can be utilised rather than a moving average.

From the scaled flow rate, two shape factors relating to the determination of partial obstruction may be calculated.

Shape factor 1 is the ratio of the mean of the middle (e.g. thirty-two) scaled flow rate points to the mean overall (e.g. sixty-five) scaled flow rate points. Where this ratio is in excess of unity, the breath will be taken to be normal. Where the ratio is unity or less, the breath will be taken to be obstructed. A ratio of about 1.17 is taken as a threshold between partially obstructed and unobstructed breathing, and equates to a degree of obstruction that would permit maintenance of adequate oxygenation in a typical patient.

Shape factor 2 is calculated as the RMS deviation from unit scaled flow rate, taken over the middle (e.g. thirty two) points. An RMS deviation of about 0.2 units is taken to be normal. An RMS deviation of zero is taken to be a totally flow-limited breath. The closer the RMS deviation to zero, the breath will be taken to be more flow limited.

Shape factors 1 and 2 may be used as alternatives, or in combination. In other forms of the present technology, the number of sampled points, breaths and middle points may differ from those described above. Furthermore, the threshold values can be other than those described.

### 5.4.3.2.5 Determination of apneas and hypopneas

In one form of the present technology, the central controller 4230 executes an apnea / hypopnea determination algorithm 4325 for the determination of the presence of apneas and/or hypopneas.

In one form, the apnea / hypopnea determination algorithm 4325 receives as an input a respiratory flow rate signal *Qr* and provides as an output a flag that indicates that an apnea or a hypopnea has been detected.

In one form, an apnea will be said to have been detected when a function of respiratory flow rate *Qr* falls below a flow rate threshold for a predetermined period of time. The function may determine a peak flow rate, a relatively short-term mean flow rate, or a flow rate intermediate of relatively short-term mean and peak flow rate, for example an RMS flow rate. The flow rate threshold may be a relatively long-term measure of flow rate.

In one form, a hypopnea will be said to have been detected when a function of respiratory flow rate *Qr* falls below a second flow rate threshold for a predetermined period of time. The function may determine a peak flow, a relatively short-term mean flow rate, or a flow rate intermediate of relatively short-term mean and peak flow rate, for example an RMS flow rate. The second flow rate threshold may be a relatively long-term measure of flow rate. The second flow rate threshold is greater than the flow rate threshold used to detect apneas.

### 5.4.3.2.6 Determination of snore

In one form of the present technology, the central controller 4230 executes one or more snore determination algorithms 4326 for the determination of the extent of snore.

In one form, the snore determination algorithm 4326 receives as an input a respiratory flow rate signal $Qr$ and provides as an output a metric of the extent to which snoring is present.

The snore determination algorithm 4326 may comprise the step of determining the intensity of the flow rate signal in the range of 30-300 Hz. Further, the snore determination algorithm 4326 may comprise a step of filtering the respiratory flow rate signal $Qr$ to reduce background noise, e.g., the sound of airflow in the system from the blower.

### 5.4.3.2.7 Determination of airway patency

In one form of the present technology, the central controller 4230 executes one or more airway patency determination algorithms 4327 for the determination of the extent of airway patency.

In one form, the airway patency determination algorithm 4327 receives as an input a respiratory flow rate signal $Qr,$ and determines the power of the signal in the frequency range of about 0.75 Hz and about 3 Hz. The presence of a peak in this frequency range is taken to indicate an open airway. The absence of a peak is taken to be an indication of a closed airway.

In one form, the frequency range within which the peak is sought is the frequency of a small forced oscillation in the treatment pressure $Pt.$ In one implementation, the forced oscillation is of frequency 2 Hz with amplitude about 1 cmH$_2$O.

In one form, airway patency determination algorithm 4327 receives as an input a respiratory flow rate signal $Qr,$ and determines the presence or absence of a cardiogenic signal. The absence of a cardiogenic signal is taken to be an indication of a closed airway.

### 5.4.3.2.8 Determination of target ventilation

In one form of the present technology, the central controller 4230 takes as input the measure of current ventilation, $Vent,$ and executes one or more target ventilation determination algorithms 4328 for the determination of a target value $Vtgt$ for the measure of ventilation.

In some forms of the present technology, there is no target ventilation determination algorithm 4328, and the target value $Vtgt$ is predetermined, for example by hard-coding during configuration of the RPT device 4000 or by manual entry through the input device 4220.

In other forms of the present technology, such as adaptive servo-ventilation (ASV), the target ventilation determination algorithm 4328 computes a target value $Vtgt$ from a value $Vtyp$ indicative of the typical recent ventilation of the patient.

In some forms of adaptive servo-ventilation, the target ventilation $Vtgt$ is computed as a high proportion of, but less than, the typical recent ventilation $Vtyp.$ The high proportion in such forms may be in the range (80%, 100%), or (85%, 95%), or (87%, 92%).

In other forms of adaptive servo-ventilation, the target ventilation $Vtgt$ is computed as a slightly greater than unity multiple of the typical recent ventilation $Vtyp.$

The typical recent ventilation $Vtyp$ is the value around which the distribution of the measure of current ventilation $Vent$ over multiple time instants over some predetermined timescale tends to cluster, that is, a measure of the central tendency of the measure of current ventilation over recent history. In one implementation of the target ventilation determination algorithm 4328, the recent history is of the order of several minutes, but in any case should be longer than the timescale of Cheyne-Stokes waxing and waning cycles. The target ventilation determination algorithm 4328 may use any of the variety of well-known measures of central tendency to determine the typical recent ventilation $Vtyp$ from the measure of current ventilation, $Vent.$ One such measure is the output of a low-pass filter on the measure of current ventilation $Vent,$ with time constant equal to one hundred seconds.

### 5.4.3.2.9 Determination of therapy parameters

In some forms of the present technology, the central controller 4230 executes one or more therapy parameter determination algorithms 4329 for the determination of one or more therapy parameters using the values returned by one or more of the other algorithms in the therapy engine module 4320.

In one form of the present technology, the therapy parameter is an instantaneous treatment pressure $Pt.$ In one implementation of this form, the therapy parameter determination algorithm 4329 determines the treatment pressure *Pt* using the equation

$$Pt = A\Pi(\Phi, t) + P_0 \qquad\qquad (1)$$

where:

- A is the amplitude,
- $\Pi(\Phi, t)$ is the waveform template value (in the range 0 to 1) at the current value $\Phi$ of phase and t of time, and
- $P_0$ is a base pressure.

If the waveform determination algorithm 4322 provides the waveform template $\Pi(\Phi, t)$ as a lookup table of values $\Pi$ indexed by phase $\Phi$, the therapy parameter determination algorithm 4329 applies equation (1) by locating the nearest lookup table entry to the current value $\Phi$ of phase returned by the phase determination algorithm 4321, or by interpolation between the two entries straddling the current value $\Phi$ of phase.

The values of the amplitude *A* and the base pressure $P_0$ may be set by the therapy parameter determination algorithm 4329 depending on the chosen respiratory pressure therapy mode in the manner described below.

### 5.4.3.3 Therapy Control module

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the present technology, the therapy parameter is a treatment pressure *Pt,* and the therapy control module 4330 controls the pressure generator 4140 to deliver a flow of air whose interface pressure *Pm* at the patient interface 3000 or 3800 is equal to the treatment pressure *Pt.*

### 5.4.3.4 Detection of fault conditions

In one form of the present technology, the central controller 4230 executes one or more methods 4340 for the detection of fault conditions. The fault conditions detected by the one or more methods 4340 may include at least one of the following:

- Power failure (no power, or insufficient power)
- Transducer fault detection
- Failure to detect the presence of a component
- Operating parameters outside recommended ranges (e.g. pressure, flow rate, temperature, $PaO_2$)
- Failure of a test alarm to generate a detectable alarm signal.

Upon detection of the fault condition, the corresponding algorithm 4340 signals the presence of the fault by one or more of the following:

- Initiation of an audible, visual &/or kinetic (e.g. vibrating) alarm
- Sending a message to an external device
- Logging of the incident

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000 or 3800.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349.

### 5.5.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3000 or 3800.

5.6 RESPIRATORY FLOW THERAPY MODES

In some versions, the RPT may be configured with a flow control loop, such as with an estimate flow signal, to provide respiratory therapies with an interface to the patient's airways that is 'open' (unsealed). The respiratory therapy may supplement the patient's own spontaneous breathing with a controlled flow of conditioned or enriched gas. In one example, High Flow therapy (HFT) in controlled an entrance to the airway through an unsealed or open patient interface at a "treatment flow rate" that is held approximately constant throughout the respiratory cycle. The treatment flow rate is nominally set to exceed the patient's peak inspiratory flow rate. As an alternative to constant flow rate, the treatment flow rate may follow a profile that varies over the respiratory cycle.

6 HUMIDIFIER

### 6.1.1 Humidifier overview

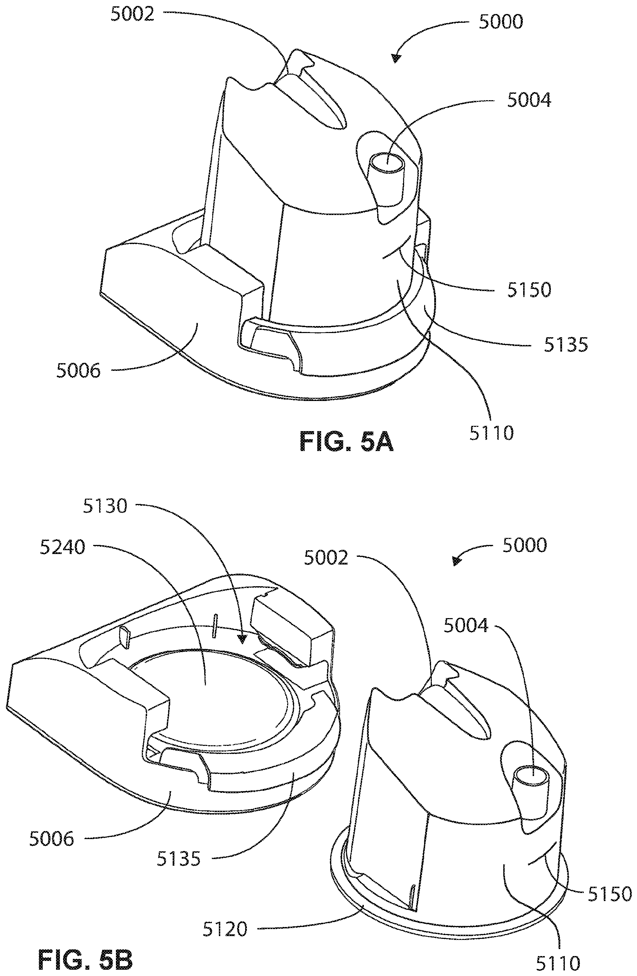

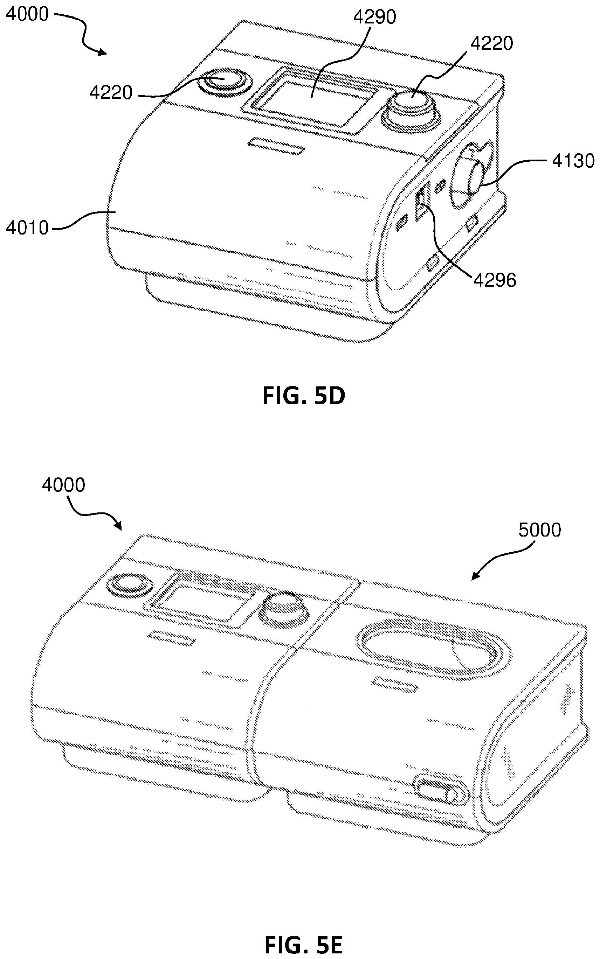

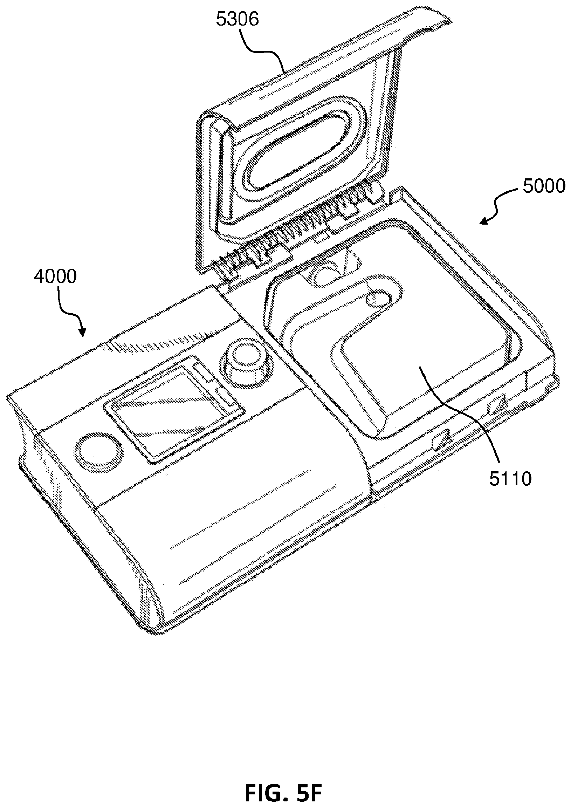

Respiratory humidifiers are available in many forms and may be a standalone device that is coupled to an RPT device via an air circuit, is integrated with the RPT device or configured to be directly coupled to the relevant RPT device. While known passive humidifiers can provide some relief, generally a heated humidifier may be used to provide sufficient humidity and temperature to the air so that the patient will be comfortable. In examples, humidifiers comprise a water reservoir or tub having a capacity of several hundred milliliters (ml), a heating element for heating the water in the reservoir, a control to enable the level of humidification to be varied, a gas inlet to receive gas from the flow generator or RPT device, and a gas outlet adapted to be connected to an air circuit that delivers the humidified gas to the patient interface.
Heated passover humidification is one common form of humidification used with an RPT device. In such humidifiers the heating element may be incorporated in a heater plate which sits under, and is in thermal contact with, the water tub. Thus, heat is transferred from the heater plate to the water reservoir primarily by conduction. The air flow from the RPT device passes over the heated water in the water tub resulting in water vapour being taken up by the air flow. The ResMed H4i™ and H5i™ Humidifiers are examples of such heated passover humidifiers that are used in combination with ResMed S8 and S9 CPAP devices respectively.
Other humidifiers may also be used such as a bubble or diffuser humidifier, a jet humidifier or a wicking humidifier. In a bubble or diffuser humidifier the air is conducted below the surface of the water and allowed to bubble back to the top. A jet humidifier produces an aerosol of water and baffles or filters may be used so that the particles are either removed or evaporated before leaving the humidifier. A wicking humidifier uses a water absorbing material, such as sponge or paper, to absorb water by capillary action. The water absorbing material is placed within or adjacent at least a portion of the air flow path to allow evaporation of the water in the absorbing material to be taken up into the air flow.
An alternative form of humidification is provided by the ResMed HumiCare™ D900 humidifier that uses a CounterStream™ technology that directs the air flow over a large surface area in a first direction whilst supplying heated water to the large surface area in a second opposite direction. The ResMed HumiCare™ D900 humidifier may be used with a range of invasive and non-invasive ventilators.
In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.
The humidifier 5000 may comprise a humidifier reservoir 5110, a humidifier inlet 5002 to receive a flow of air, and a humidifier outlet 5004 to deliver a humidified flow of air. In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 6.1.2 Humidifier components

### 6.1.2.1 Water reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 5A and Fig. 5B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

### 6.1.2.2 Conductive portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

### 6.1.2.3 Humidifier reservoir dock

In one form, the humidifier 5000 may comprise a humidifier reservoir dock 5130 (as shown in Fig. 5B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

### 6.1.2.4 Water level indicator

The humidifier reservoir 5110 may comprise a water level indicator 5150 as shown in Fig. 5A-5B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

### 6.1.2.5 Humidifier transducer(s)

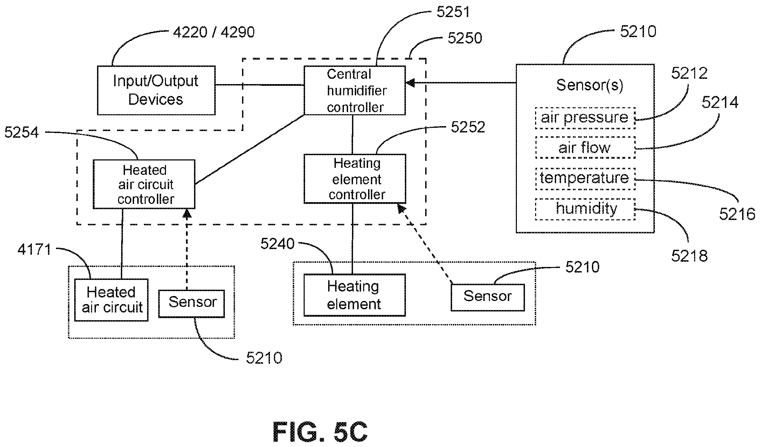

The humidifier 5000 may comprise one or more humidifier transducers (sensors) 5210 instead of, or in addition to, transducers 4270 described above. Humidifier transducers 5210 may include one or more of an air pressure sensor 5212, an *air* flow rate transducer 5214, a temperature sensor 5216, or a humidity sensor 5218 as shown in Fig. 5C. A humidifier transducer 5210 may produce one or more output signals which may be communicated to a controller such as the central controller 4230 and/or the humidifier controller 5250. In some forms, a humidifier transducer may be located externally to the humidifier 5000 (such as in the *air* circuit 4170) while communicating the output signal to the controller.

#### 6.1.2.5.1 Pressure transducer

One or more pressure transducers 5212 may be provided to the humidifier 5000 in addition to, or instead of, a pressure sensor 4272 provided in the RPT device 4000.

#### 6.1.2.5.2 Flow rate transducer

One or more flow rate transducers 5214 may be provided to the humidifier 5000 in addition to, or instead of, a flow rate sensor 4274 provided in the RPT device 4000.

### 6.1.2.5.3 Temperature transducer

The humidifier 5000 may comprise one or more temperature transducers 5216. The one or more temperature transducers 5216 may be configured to measure one or more temperatures such as of the heating element 5240 and/or of the flow of *air* downstream of the humidifier outlet 5004. In some forms, the humidifier 5000 may further comprise a temperature sensor 5216 to detect the temperature of the ambient air.

### 6.1.2.5.4 Humidity transducer

In one form, the humidifier 5000 may comprise one or more humidity sensors 5218 to detect a humidity of a gas, such as the ambient air. The humidity sensor 5218 may be placed towards the humidifier outlet 5004 in some forms to measure a humidity of the gas delivered from the humidifier 5000. The humidity sensor may be an absolute humidity sensor or a relative humidity sensor.

### 6.1.2.6 Heating element

A heating element 5240 may be provided to the humidifier 5000 in some cases to provide a heat input to one or more of the volume of water in the humidifier reservoir 5110 and/or to the flow of air. The heating element 5240 may comprise a heat generating component such as an electrically resistive heating track. One suitable example of a heating element 5240 is a layered heating element such as one described in the PCT Patent Application Publication No. WO 2012/171072.
In some forms, the heating element 5240 may be provided in the humidifier base 5006 where heat may be provided to the humidifier reservoir 5110 primarily by conduction as shown in Fig. 5B.

### 6.1.2.7 Humidifier controller

According to one arrangement of the present technology, a humidifier 5000 may comprise a humidifier controller 5250 as shown in Fig. 5C. In one form, the humidifier controller 5250 may be a part of the central controller 4230. In another form, the humidifier controller 5250 may be a separate controller, which may be in communication with the central controller 4230.
In one form, the humidifier controller 5250 may receive as inputs measures of properties (such as temperature, humidity, pressure and/or flow rate), for example of the flow of air, the water in the reservoir 5110 and/or the humidifier 5000. The humidifier controller 5250 may also be configured to execute or implement humidifier algorithms and/or deliver one or more output signals.
As shown in Fig. 5C, the humidifier controller 5250 may comprise one or more controllers, such as a central humidifier controller 5251, a heated air circuit controller 5254 configured to control the temperature of a heated air circuit 4171 and/or a heating element controller 5252 configured to control the temperature of a heating element 5240.

### 6.1.2.8 Releasable connection to RPT device

Figs. 5D to 5F show a further RPT device 4000 according to another form of the present technology. The device includes an external housing 4010 which contains a pressure generator 4140 (not shown) for producing a flow of breathable gas at a positive pressure. For example, the pressure generator 4140 may be a controllable blower. The device 4000 also comprises input devices 4220 in the form of buttons, and dials, and output devices 4290 in the form of a display. The input devices 4220 and output devices 4290 together provide the user with a user interface for controlling and configuring the device 4000.
The device 4000 also comprises an outlet 4130 for supplying the flow of breathable gas to one or more downstream devices which are fluidly connected to the device 4000. In one form of the technology, the device 4000 is configured to releasably connect to a releasable component in the form of humidifier 5000. The releasable connection between the device 4000 and the humidifier 5000 may be provided by a mechanical connection mechanism which, when engaged, aligns the device 4000 and humidifier 5000 in such a way that an outlet 4130 of the device 4000 is fluidly connected with an inlet 5002 of the humidifier 5000 and one or more electrical connections 4296 of the device 4000 are electrically connected to corresponding electrical connections on the humidifier 5000.
The humidifier has a humidifier housing 5300 which is separate to the external housing 4010 of the device 4000. The humidifier housing 5300 contains a water reservoir 5110 and a heating element 5240 (not shown) configured to heat the water contained in the water reservoir 5110 to thereby facilitate humidification of the flow of breathable gas. In this example, the water reservoir 5110 may be removed from the humidifier housing 5300 by opening the lid or top cover 5306 of the humidifier 5000. In alternative examples, the water reservoir 5110 may be removed through an end or side of the humidifier 5000.

## 6.1.3 Liquid trap

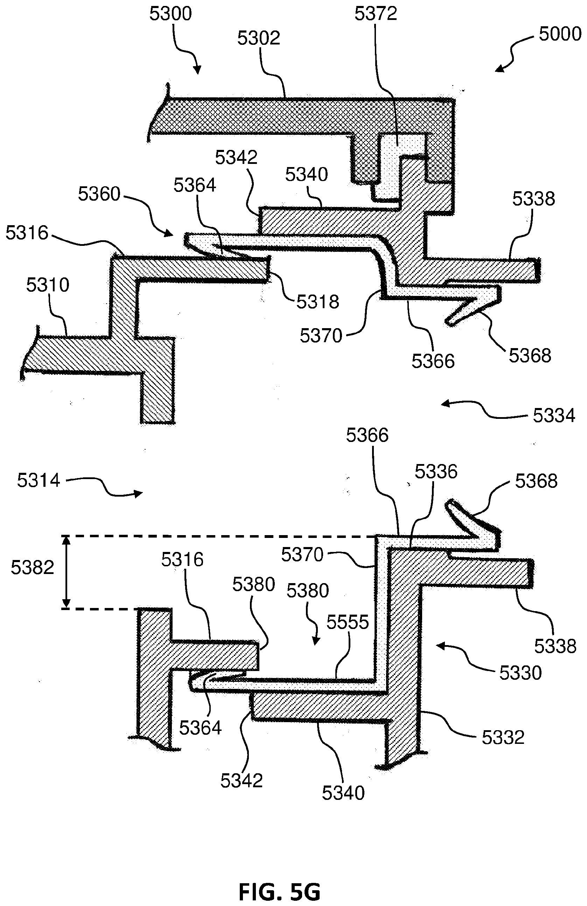

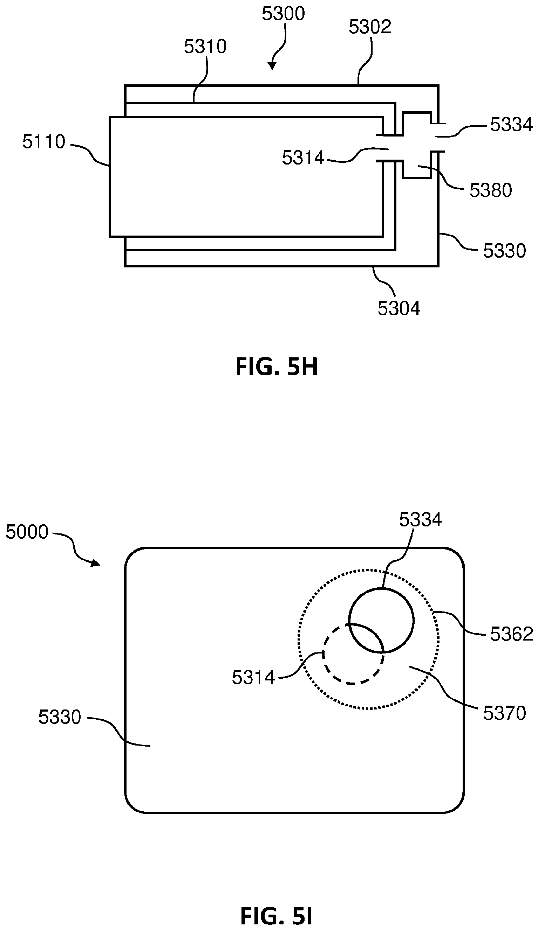

According to one aspect of the present technology, as shown in Fig. 5G, a humidifier 5000 may have a body comprising an external housing 5300. Referring to Fig. 5H, the housing 5300 may be formed in two parts, an upper portion 5302 and a lower portion 5304. The body of humidifier 5000 further comprises a chassis 5310.

Reference to a chassis herein should be understood to mean a supporting frame of a structure - i.e. a structural element configured to support one or more other components, more particularly one or more internal components of the humidifier 5000. Reference to a housing should be understood to mean an element that covers or protects other components of a structure. In the example of Fig. 5G to 5I, the housing 5300 is provided to at least partially cover or protect the chassis 5310. In alternative examples, the humidifier 5000 may comprise a housing 5310 configured to act as a chassis 5310. In alternative examples, the humidifier 5000 may comprise a chassis 5310 without a separate housing *per se.*

In examples, the humidifier 5000 comprises a removable container in the form of water reservoir 5110. The chassis 5310 is configured to locate and support the removable reservoir 5110 in use. In the example shown in Fig. 5H, the reservoir 5110 is inserted and removed from an end of the humidifier. In alternative examples, the reservoir 5110 may be removed from a side of the humidifier 5000 (i.e. laterally), or from above or below (i.e. vertically). PCT Patent Application Publication No. WO 2018/094452 AI describes exemplary arrangements for a humidifier having a removable water reservoir.

In alternative examples, the chassis 5310 may comprise a chamber which functions as the water reservoir 5110 - i.e. is integrated with the chassis 5310 rather than being removable.

According to one aspect of the present technology, as shown in Fig. 5G, the humidifier 5000 comprises a closure element in the form of an end cap 5330. In this example the end cap 5330 is configured to seal against the housing 5300 and the chassis 5310, as described further below.

In examples, the end cap 5330 has a main cap wall 5332 comprising an air inlet port 5334 configured to be connected to a source of a flow of air at positive pressure, for example RPT device 4000. The air inlet port 5334 comprises an inner surface 5336 and an external annular port wall 5338 extending from the main cap wall 5332. The end cap 5330 further comprises an inner peripheral wall 5340 surrounding the air inlet port 5334 and extending towards the chassis 5310. In this example, the inner peripheral wall 5340 is annular.

In examples, the chassis 5310 has a vertical chassis wall 5312 comprising a chamber inlet port 5314. In the example of Fig. 5G to 5I, the chamber inlet port 5314 pneumatically connects to an inlet port of the reservoir 5510. A chassis peripheral wall 5316 surrounds the chamber inlet port 5314 and extends towards the end cap 5330. In this example, the chassis peripheral wall 5316 is annular.

In examples, a free end 5318 of the chassis peripheral wall 5316 overlaps a free end 5342 of the inner peripheral wall 5340. A resilient seal 5360 is provided between the chassis peripheral wall 5316 and the inner peripheral wall 5340 to seal the space between the air inlet port 5334 and the chamber inlet port 5314. This provides a gas flow path between the air inlet port 5334 and the chamber inlet port 5314, and forms a liquid trap 5380 for retention of a volume of water spilled through the chamber inlet port 5314.

There are various circumstances in which water may pass through the chamber inlet port 5314 from the reservoir 5110, including knocking of the humidifier 5000 or a stand on which it sits to produce a sloshing effect, or tipping of the humidifier 5000 as it is shifted or re-oriented. The liquid trap 5380 is provided to retain a volume of this spilled water to reduce the likelihood of water reaching other components of the system upstream, more particularly the RPT device 4000.

Further, the liquid trap 5380 protects the space between the chassis 5310 and the end cap 5300 below the liquid trap 5380. Humidifier components which might otherwise be susceptible to water damage (for example electrical components) may be positioned in this space.

## 6.1.3.1 Resilient seal

As shown in Fig. 5G, the resilient seal 5360 comprises a liquid trap portion 5362 positioned between the chassis peripheral wall 5316 and the inner peripheral wall 5340. The liquid trap portion 5362 is tubular in form, and covers an interior surface of the inner peripheral wall 5340. The liquid trap seal portion 5362 further comprises a first lip 5364 projecting radially inwardly to bear against an external surface of the chassis peripheral wall 5316. The first lip 5364 projects at an angle back towards the end cap 5330.

The resilient seal 5360 further comprises an air inlet port portion 5366 provided on the inner surface 5336 of the air inlet port 5334. The air inlet port portion 5366 comprises a second lip 5368 projecting radially inwardly at an angle back towards the chassis 5310. The resilient seal 5360 further comprises a flange portion 5370 between the air inlet port portion 5366 and the liquid trap portion 5362. In the example shown, the flange portion 5370 covers an inner surface of the main cap wall 5332 between the air inlet port 5334 and the inner peripheral wall 5340.

In examples each of the inner peripheral wall 5340 and the chassis peripheral wall 5316 are circular - i.e. generally take the form of cylindrical tubes. The liquid trap seal portion 5362 of the resilient seal 5360 is complementary in shape - i.e. is also a cylindrical tube. This circular geometry may assist with forming the seal therebetween.

In the example shown, the resilient seal 5360 comprises a peripheral seal portion 5372 formed as a unitary part. The peripheral seal portion 5372 is provided between the housing 5300 and the end cap 5330 to seal at least a portion of a periphery of the end cap 5330.

In examples, the resilient seal may be made of a silicone, a thermoplastic elastomer, or any other suitable resilient material.

In examples, the resilient seal 5360 may be overmolded to the end cap 5530. In alternative examples the resilient seal 5360 and the end cap 5530 may be manufactured separately and subsequently assembled.

In an alternate example, a first resilient seal may be provided between the chassis peripheral wall 5316 and the inner peripheral wall 5340, and one or more further resilient seals may be used to provide the functions of the air inlet port portion 5366 and/or the peripheral seal portion 5372. For example, the first resilient seal may be an O-ring.

In alternative examples, the chassis peripheral wall 5316 may seal directly against the inner peripheral wall 5340. For example, an engineering fit between the chassis peripheral wall 5316 and the inner peripheral wall 5340 may be used to produce a seal.

### 6.1.3.2 Relative positioning of air inlet port and chamber inlet port

In addition to preventing spilled water from reaching air inlet port 5334, it may be desirable to return at least a portion of the trapped water to the reservoir 5110 when the humidifier 5000 is returned to an intended working orientation.

As shown in Fig. 5G to 5I, the chamber inlet port 5314 is positioned relative to the air inlet port 5334 such that when the humidifier is in an intended working orientation, a lowest point of the chamber inlet port 5314 is inferior to a lowest point of the air inlet port 5334 - i.e. there is a vertical separation 5382 between these points.

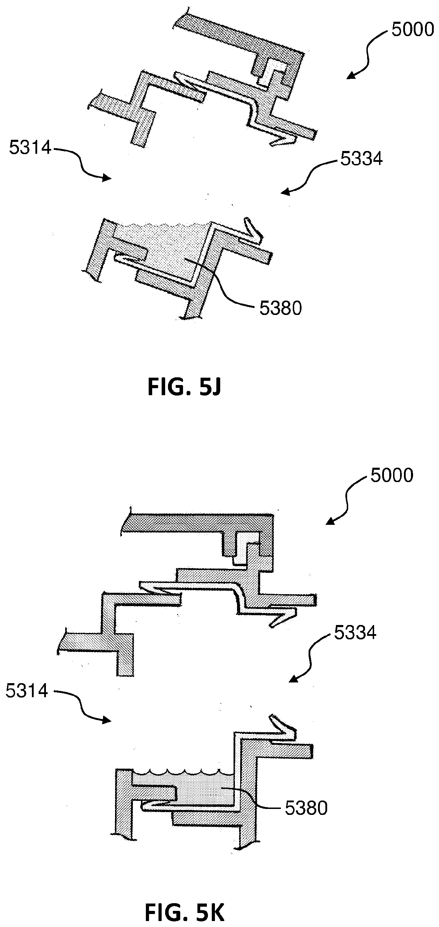

Referring to Fig. 5J, when the humidifier 5000 is tipped such that water is displaced through chamber inlet port 5314, the liquid trap 5380 can retain a volume of water below a level between the chamber inlet port 5314 and air inlet port 5334. In the example shown in Fig. 5J, the liquid trap is configured to accommodate a tip angle of substantially 20 degrees from level. It will be appreciated that the volume of water to be retained will be somewhat dependent on the total capacity and configuration of the humidifier 5000, but in this example the liquid trap 5380 may be configured to capture a volume of about 40 to 70 ml of water, more particularly about 50 ml of water. When the humidifier 5000 is returned to its intended working orientation (i.e. a substantially level orientation), as shown in Fig. 5K, the vertical separation 5382 ensures that at least some water may drain from the liquid trap 5380 back through the chamber inlet port 5314.

While the liquid trap 5380 may retain a small volume of the spilled water, as shown in Fig. 5K, this will be less than the total capacity of the liquid trap 5380. It is envisaged that this relatively small volume of water may be readily evaporated by airflow through the liquid trap 5380 in use.

In examples, as shown in Fig. 5I a centre of the chamber inlet port 5314 is laterally offset from a centre of the air inlet port 5334. This offset may assist in accommodation of other components of the humidifier 5000 or RPT device 4000. This offset may also be used to reduce the likelihood of spilling through the air inlet port 5334 when the humidifier 5000 is tipped in certain directions, based on expected orientation in use relative to environmental features such as the edge of a bedside table from which the device may be knocked accidentally.

### 6.1.3.3 Superior and lateral portions

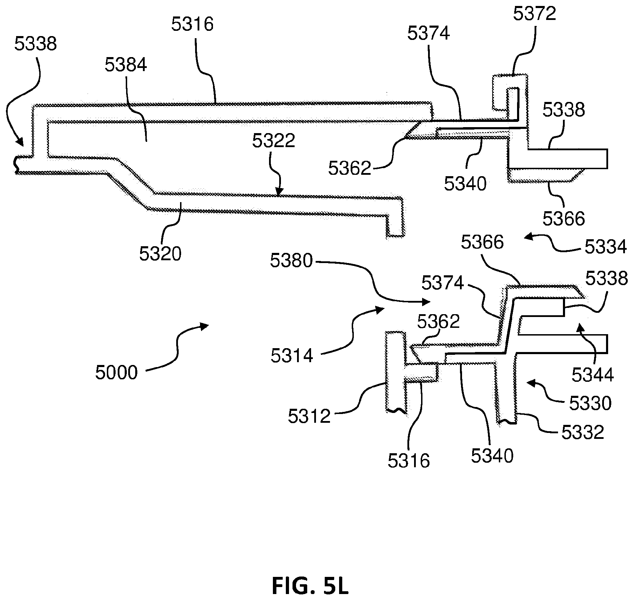

According to one aspect of the present technology, as shown in Fig. 5L, in examples the liquid trap 5380 comprises a superior portion 5384 above the reservoir 5110 (not shown in Fig. 5L) when the humidifier 5000 is in an in-use orientation. This superior portion 5384 provides additional volume to the liquid trap 5380 for capture of water being spilled from the reservoir 5110 in the event of knocking or tipping of the humidifier 5000, particularly an event that results in the humidifier being tipped upside down from its intended orientation in use. In this example, it is envisaged that the liquid trap 5380 may accommodate a volume of about 60 ml of spilled water.

In examples, the chassis 5310 comprises an inner chassis wall portion 5320, providing at least a portion of a supporting structure for the reservoir 5110 (or the reservoir itself in certain examples). In the example of Fig. 5L, a superior portion of the chassis peripheral wall 5316 extends towards the end cap 5330 from a position on the inner chassis wall portion 5320 away from the vertical chassis wall 5312. The volume between the superior portion of the chassis peripheral wall 5316 and the inner chassis wall portion 5320 provides the superior portion 5384 of the liquid trap 5380.

In examples, the superior portion 5384 is configured such that liquid drains from the superior portion 5384 into an inferior portion of the liquid trap 5380 (for example, where the chamber inlet port 5314 opens into the liquid trap 5380), when the humidifier 5000 is returned to its intended working orientation (i.e. a substantially level orientation). Draining of the liquid reduces the likelihood of pooling of the liquid and bacteria contamination. In examples, a superior facing surface 5322 of the inner chassis wall portion 5320 is inclined to encourage draining - as shown in Fig. 5L. In alternative examples, the superior facing surface 5322 may comprise one or more channels through which draining occurs.

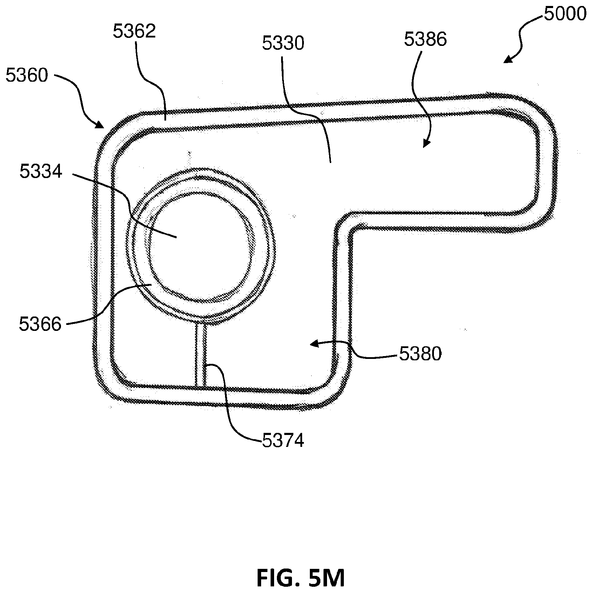

According to one aspect of the present technology, as shown in Fig. 5M, in examples the liquid trap 5380 comprises a lateral portion 5386. The lateral portion 5386 extends laterally from the portion of the liquid trap 5380 surrounding the chamber inlet port 5314 (i.e. to the side of the chamber inlet port 5314 and/or the air inlet port 5334). The lateral portion 5386 provides additional volume to the liquid trap 5380 for capture of water being spilled from the reservoir 5110 in the event of knocking or tipping of the humidifier 5000. The height of the lateral portion 5386 is less than the greatest height of the liquid trap 5380, where height is the dimension in the superior-inferior direction when the humidifier 5000 is in an in-use orientation. In examples, the lateral portion 5386 may extend from the superior portion 5384 shown in Fig. 5L.

In examples, the lateral portion 5386 is configured such that liquid drains into an inferior portion of the liquid trap 5380 (for example, where the chamber inlet port 5314 opens into the liquid trap 5380) when the humidifier 5000 is returned to its intended working orientation (i.e. a substantially level orientation). In examples, a superior facing surface of the lateral portion 5386 may be inclined, and/or include channels, to encourage draining.

In the example shown in Fig. 5L, the end cap 5330 includes a locating feature 5344 at the air inlet port 5334. In this example, the locating feature 5344 is a recess configured to receive a complementary locating feature of a connector (not shown) inserted into the air inlet port 5334, for example to connect the humidifier 5000 to a flow generator or RPT device.

In the example shown in Fig. 5L and Fig. 5M, the resilient seal 5360 does not cover the entirety of the inner surfaces of the end cap 5330 forming the liquid trap 5380. In this example the liquid trap portion 5362 of the seal 5360 is provided on the rim of the inner peripheral wall 5340 to seal against radially inwardly facing surfaces of the chassis peripheral wall 5340, and the air inlet port portion 5366 of the seal 5360 is provided at the air inlet port 5334, but the majority of the surface area of the liquid trap 5380 is provided by the walls of the end cap 5330. In this example, the liquid trap portion 5362 and the air inlet port portion 5366 are connected by one or more seal runner portions 5374 to allow for manufacture of the seal 5360 as one part. It should be appreciated that other seal features of the end cap 5330 (for example, peripheral seal portion 5372) may also be connected to one or more of the liquid trap portion 5362 and the air inlet port portion 5366.

### 6.1.3.4 Inverted receptacle

According to one aspect of the present technology, as shown in Fig. 6A, an inverted receptacle 5600 may be provided to a superior portion of the chamber inlet port 5314 when the humidifier 5000 is in an intended working orientation. The inverted receptacle 5600 comprises a closed end 5602 and an open end 5604, with a curved receptacle wall 5606 to provide a semicylindrical form. The open end 5604 intersects and aligns with the chamber inlet port 5314.

As shown in Fig. 6B, in this example the inverted receptacle extends from the chassis 5310 towards the end cap 5330. In the event of knocking or tipping of the humidifier 5000, particularly an event that results in the humidifier being tipped upside down from its intended orientation in use (i.e. inverted), water spilling through the chamber inlet port 5314 may be captured within the receptacle 5600, or at least partially diverted into the liquid trap 5380 rather than spilling directly into the air inlet port 5334.

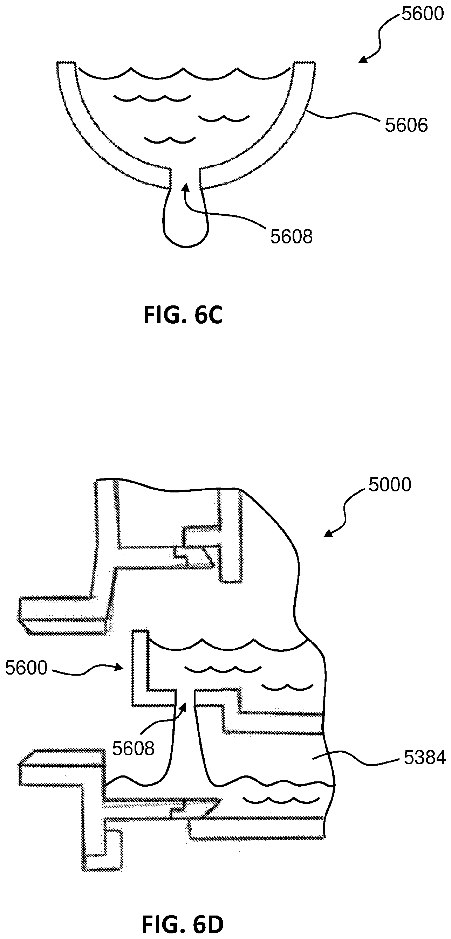

In examples, as shown in Fig. 6C and Fig. 6D the inverted receptacle 5600 comprises at least one drain hole 5608. In the example illustrated the drain hole 5608 is provided in a superior facing surface of the receptacle wall 5606 (noting that Fig. 6C and Fig. 6D show the receptacle when the humidifier 5000 has been inverted from an intended working orientation), at the apex of the curve. In use liquid may drain through the drain hole 5608 into the liquid trap 5384, and reduce the likelihood of spilling over the closed end of the receptacle 5600 towards the air inlet 5334.

### 6.1.3.5 Interior trap wall

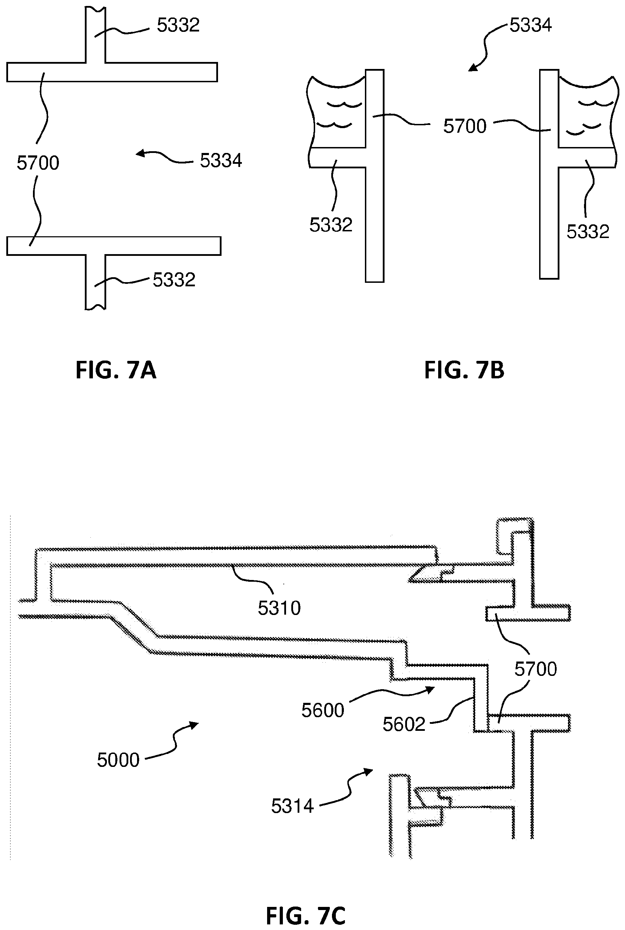

According to one aspect of the present technology, as shown in Fig. 7A and Fig. 7B, the humidifier 5000 comprises an interior trap wall 5700 surrounding the air inlet port 5334, and extending from the end cap 5330 (more particularly the main cap wall 5332) towards the chamber inlet port 5314. The volume surrounding the interior trap wall 5700 forms a catchment of the liquid trap when the humidifier 5000 is tipped on, or at least towards, its end.

In examples, as shown in Fig. 7C, an inverted receptacle 5600 extends from the chassis 5310 towards the interior trap wall 5700. In the example illustrated the inverted receptacle 5600 extends to be proximal to, and contact, the interior trap wall 5700. In doing so, in the event of liquid being spilled through the chamber inlet port 5314 when the humidifier 5000 is inverted, spilling of the liquid directly from the receptacle 5600 into the air inlet port 5334 is discouraged.

### 6.1.3.6 Baffle portions

According to one aspect of the present technology the humidifier 5000 comprises a baffle portion positioned between the air inlet port and the chamber inlet port, configured to direct liquid flowing from the chamber inlet port 5314 in a radial direction.

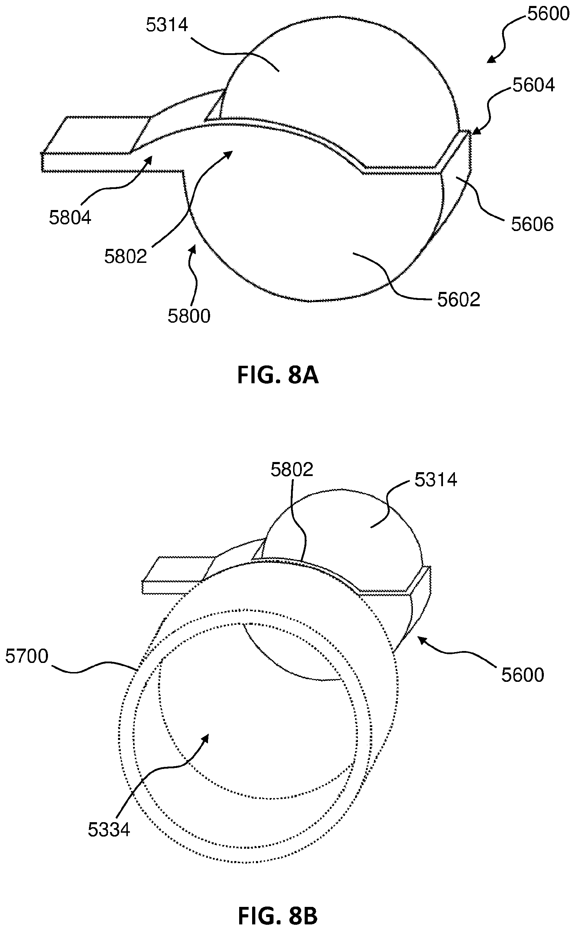

In examples, as shown in Fig. 8A and Fig. 8B, a baffle portion 5800 may form part of an inverted receptacle 5600. The closed end 5602 may include a baffle extension 5802 extending the area of the closed end 5602, shaped to block a portion of the air inlet port 5334 (as shown in Fig. 8B). The inverted receptacle 5600 further comprises a radially extending baffle portion 5804, which functions to direct liquid being spilled from the chamber inlet port 5314 away from the air inlet port 5334.

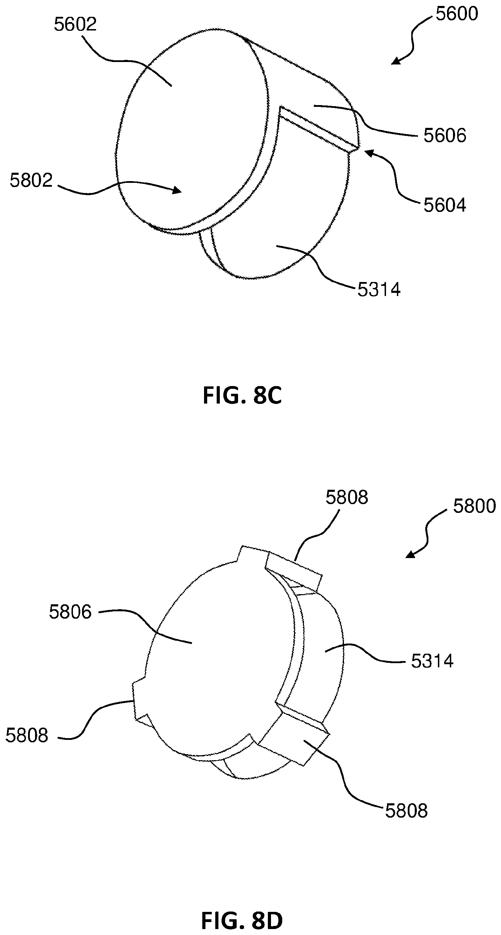

Fig. 8C shows an alternative example in which the closed end 5602 of inverted receptacle 5600 comprises a baffle extension 5802 forming a complete disc. Fig. 8D illustrates an alternative baffle portion 5800 comprising a flat upright baffle member 5806 supported by a plurality of baffle support members in the form of baffle legs 5808. The baffle legs 5808 extend from the chassis 5310, with gaps between the baffle legs 5808 and the baffle member 5806 permitting the flow of air therethrough.

### 6.1.3.7 Liquid trap partition

According to one aspect of the present technology the humidifier 5000 comprises at least one liquid trap partition 5850 providing a plurality of liquid trap catchments within the liquid trap 5380.

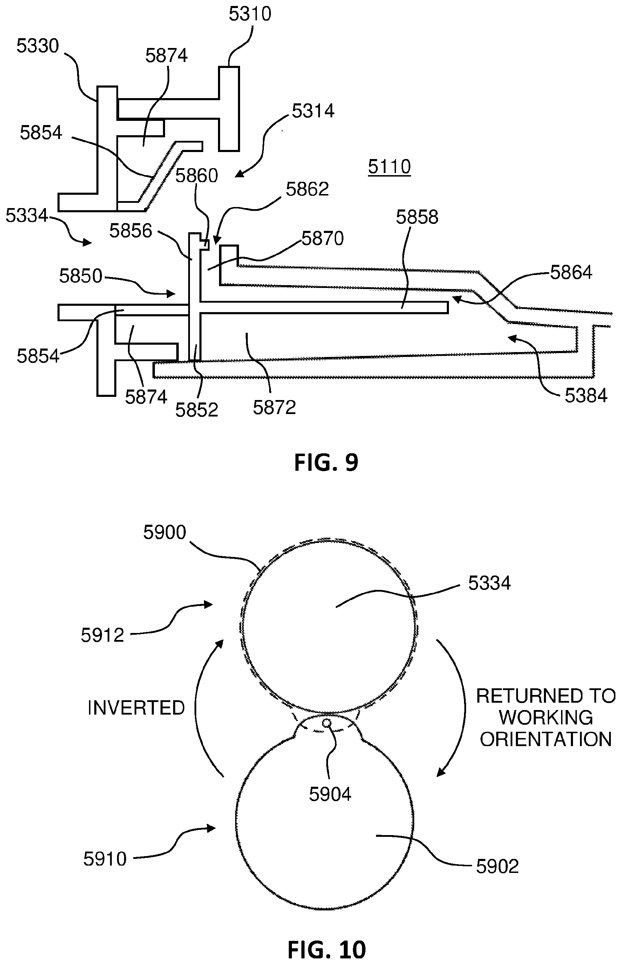

In examples, as shown in Fig. 9, the liquid trap partition 5850 comprises a first portion 5852 and a second portion 5854. In examples the first portion 5852 and the second portion 5854 are separate parts, while in alternative examples the first portion 5852 and the second portion 5854 may be a unitary part. The first portion 5852 comprises an upright intermediary wall 5856, and a dividing wall 5858 extending from the upright intermediary wall 5856 into the superior portion 5384 above the reservoir 5110 (noting that Fig. 9 shows the humidifier 5000 in an inverted orientation). A partition wall 5860 extends from the upright intermediary wall towards the chassis 5310, wherein a first gap 5862 is provided between the chamber inlet port 5314 and the partition wall 5860.

When the humidifier 5000 is in an inverted orientation, a first liquid trap catchment 5870 is provided inferior to the chamber inlet port 5314 - through the first gap 5862 and bounded by the upright intermediary wall 5856, the partition wall 5860, and dividing wall 5858. A second liquid trap catchment 5872 is provided inferior to the first liquid trap catchment 5870, with liquid running from the first liquid trap catchment 5870 along the dividing wall 5858 and through a second gap 5864 at a free end of the dividing wall 5858.

In examples the second portion 5854 forms an interior trap wall extending from the end cap 5330 towards the chamber inlet port 5314. The second portion 5854 surrounds the air inlet port 5334 to define a third liquid trap catchment 5874 (i.e. the volume around the exterior of the second portion 5854).

### 6.1.3.8 Moveable barrier

According to one aspect of the present technology, as shown in Fig. 10, the humidifier 5000 comprises a moveable barrier 5900 configured to selectively block the air inlet port 5334 when the humidifier 5000 is inverted from an intended working orientation. For completeness, it is noted that in examples a moveable barrier 5900 may be used to block the chamber inlet port 5314 - instead of, or in addition to, use of a moveable barrier 5900 to selectively block the air inlet port 5334.

The moveable barrier 5900 is configured to move between a stored position 5910 away from the air inlet port 5334 when the humidifier is in an intended working orientation, and a closed position 5912 blocking the air inlet port 5334 when the humidifier 5000 is in an inverted orientation.

In the example shown, the moveable barrier 5900 comprises a barrier body 5902 in the form of a flat plate, having a pivot 5904 (for example a pin or protrusion) positioned inferior to the air inlet port 5334. As the humidifier 5000 is tipped off-axis, gravity causes the barrier body 5902 to pivot about the pivot 5904 and fall to cover the air inlet port 5334. As the humidifier 5000 is returned to its intended working orientation, gravity causes the barrier body 5902 to pivot about the pivot 5904 and fall to away from the air inlet port 5334.

In an alternative example, the moveable barrier 5900 may comprise a hinge, such that the barrier body 5902 functions as a hinged lid. In an alternative example the moveable barrier 5900 may be configured to slide relative to the air inlet port 5334, for example sliding on one or more tracks.

### 6.1.3.9 Secondary closure element

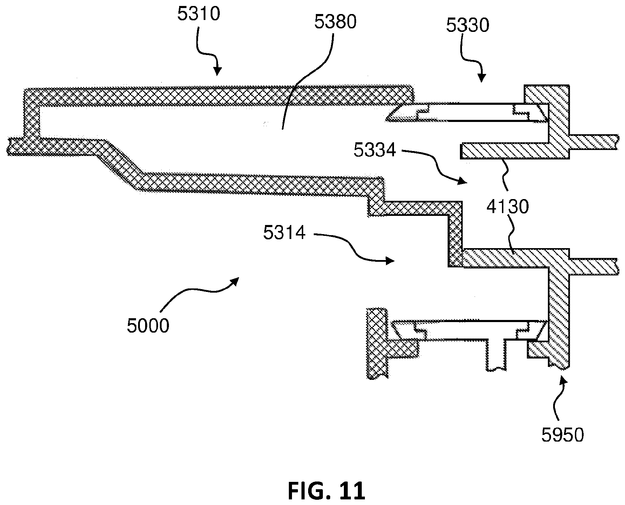

According to one aspect of the present technology, as shown in Fig. 11, the end cap 5330 may be configured to interact with a secondary closure element 5950 to provide the liquid trap 5380. For example, the secondary closure element 5950 may be a component of the RPT device 4000. In such an example, the secondary closure element 5950 may comprise outlet 4130 which acts as the air inlet port 5334.

In alternative examples, the secondary closure element 5950 may function as an interface between the RPT device 4000 and the humidifier 5000 - e.g. pneumatically connecting to the outlet 4000 and interfacing with the end cap 5330 to provide the air inlet port 5334.

It should be appreciated that, in examples, one or more features described herein as being part of the end cap 5330 may be embodied in the secondary closure element 5950, and operate cooperatively with the end cap 5330 to achieve the functionality described.

## 6.2 GLOSSARY

For the purposes of the present technology disclosure, in certain forms of the present technology, one or more of the following definitions may apply. In other forms of the present technology, alternative definitions may apply.

### 6.2.1 General

*Air*: In certain forms of the present technology, air may be taken to mean atmospheric air, and in other forms of the present technology air may be taken to mean some other combination of breathable gases, e.g. atmospheric air enriched with oxygen.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

*Continuous Positive Airway Pressure* (*CPAP*) *therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate*: The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, $Qd$, is the flow rate of air leaving the RPT device. Total flow rate, $Qt$, is the flow rate of air and any supplementary gas reaching the patient interface via the air circuit. Vent flow rate, $Qv$, is the flow rate of air leaving a vent to allow washout of exhaled gases. Leak flow rate, $Ql$, is the flow rate of leak from a patient interface system or elsewhere. Respiratory flow rate, $Qr$, is the flow rate of air that is received into the patient's respiratory system.

*Flow therapy*: Respiratory therapy comprising the delivery of a flow of air to an entrance to the airways at a controlled flow rate referred to as the treatment flow rate that is typically positive throughout the patient's breathing cycle.

*Humidifier*: The word humidifier will be taken to mean a humidifying apparatus constructed and arranged, or configured with a physical structure to be capable of providing a therapeutically beneficial amount of water ($H_2O$) vapour to a flow of air to ameliorate a medical respiratory condition of a patient.

*Leak*: The word leak will be taken to be an unintended flow of air. In one example, leak may occur as the result of an incomplete seal between a mask and a patient's face. In another example leak may occur in a swivel elbow to the ambient.

*Noise, conducted* (*acoustic*): Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated* (*acoustic*): Radiated noise in the present document refers to noise which is carried to the patient by the ambient air. In one form, radiated noise may be quantified by measuring sound power/pressure levels of the object in question according to ISO 3744.

*Noise, vent* (*acoustic*): Vent noise in the present document refers to noise which is generated by the flow of air through any vents such as vent holes of the patient interface.

*Patient*: A person, whether or not they are suffering from a respiratory condition.

*Pressure*: Force per unit area. Pressure may be expressed in a range of units, including $cmH_2O$, g-f/cm$^2$ and hectopascal. 1 $cmH_2O$ is equal to 1 g-f/cm$^2$ and is approximately 0.98 hectopascal (1 hectopascal = 100 Pa = 100 N/m$^2$ = 1 millibar ~ 0.001 atm). In this specification, unless otherwise stated, pressure is given in units of $cmH_2O$.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy* (*RPT*): The application of a supply of air to an entrance to the airways at a treatment pressure that is typically positive with respect to atmosphere.

*Ventilator*: A mechanical device that provides pressure support to a patient to perform some or all of the work of breathing.

### 6.2.1.1 Materials

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate*: a thermoplastic polymer of Bisphenol-A Carbonate.

### 6.2.1.2 Mechanical properties

*Resilience*: Ability of a material to absorb energy when deformed elastically and to release the energy upon unloading.

*Resilient*: Will release substantially all of the energy when unloaded. Includes e.g. certain silicones, and thermoplastic elastomers.

*Hardness*: The ability of a material *per se* to resist deformation (e.g. described by a Young's Modulus, or an indentation *hardness* scale measured on a standardised sample size).

- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness* (*or rigidity*) *of a structure or component*: The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component*: A structure or component that will change shape, e.g. bend, when caused to support its own weight, within a relatively short period of time such as 1 second.

*Rigid structure or component*: A structure or component that will not substantially change shape when subject to the loads typically encountered in use. An example of such a use may be setting up and maintaining a patient interface in sealing relationship with an entrance to a patient's airways, e.g. at a load of approximately 20 to 30 $cmH_2O$ pressure.

As an example, an I-beam may comprise a different bending stiffness (resistance to a bending load) in a first direction in comparison to a second, orthogonal direction. In another example, a structure or component may be floppy in a first direction and rigid in a second direction.

### 6.2.2 Respiratory cycle

*Apnea*: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate*: The rate of spontaneous respiration of a patient, usually measured in breaths per minute.

*Duty cycle*: The ratio of inhalation time, *Ti* to total breath time, *Ttot.*

*Effort (breathing)*: The work done by a spontaneously breathing person attempting to breathe.

*Expiratory portion of a breathing cycle*: The period from the start of expiratory flow to the start of inspiratory flow.

*Flow limitation*: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:

(i) *Flattened*: Having a rise followed by a relatively flat portion, followed by a fall.

(ii) *M-shaped*: Having two local peaks, one at the leading edge, and one at the trailing edge, and a relatively flat portion between the two peaks.

(iii) *Chair-shaped*: Having a single local peak, the peak being at the leading edge, followed by a relatively flat portion.

(iv) *Reverse-chair shaped*: Having a relatively flat portion followed by single local peak, the peak being at the trailing edge.

*Hypopnea*: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:

(i) a 30% reduction in patient breathing for at least 10 seconds plus an associated 4% desaturation; or

(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle*: The period from the start of inspiratory flow to the start of expiratory flow will be taken to be the inspiratory portion of a breathing cycle.

*Patency (airway)*: The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP)*: The pressure above atmosphere in the lungs that exists at the end of expiration.

*Peak flow rate (Qpeak)*: The maximum value of flow rate during the inspiratory portion of the respiratory flow waveform.

*Respiratory flow rate,* patient *airflow rate, respiratory airflow rate (Qr)*: These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt)*: The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti)*: The duration of the inspiratory portion of the respiratory flow rate waveform.

*(exhalation) Time (Te)*: The duration of the expiratory portion of the respiratory flow rate waveform.

*(total) Time (Ttot)*: The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO)*: includes both partial and total upper airway obstruction. This may be associated with a state of flow limitation, in which the flow rate increases only slightly or may even decrease as the pressure difference across the upper airway increases (Starling resistor behaviour).

*Ventilation (Vent)*: A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 6.2.3 Ventilation

*Adaptive Servo-Ventilator (ASV)*: A servo-ventilator that has a changeable, rather than fixed target ventilation. The changeable target ventilation may be learned from some characteristic of the patient, for example, a respiratory characteristic of the patient.

*Backup rate*: A parameter of a ventilator that establishes the minimum breathing rate (typically in number of breaths per minute) that the ventilator will deliver to the patient, if not triggered by spontaneous respiratory effort.

*Cycled*: The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP)*: a base pressure, to which a pressure varying within the breath is added to produce the desired interface pressure which the ventilator will attempt to achieve at a given time.

*End expiratory pressure (EEP)*: Desired interface pressure which the ventilator will attempt to achieve at the end of the expiratory portion of the breath. If the pressure waveform template $\Pi(\Phi)$ is zero-valued at the end of expiration, i.e. $\Pi(\Phi) = 0$ when $\Phi = 1$, the EEP is equal to the EPAP.

*Inspiratory positive airway pressure (IPAP)*: Maximum desired interface pressure which the ventilator will attempt to achieve during the inspiratory portion of the breath.

*Pressure support*: A number that is indicative of the increase in pressure during ventilator inspiration over that during ventilator expiration, and generally means the difference in pressure between the maximum value during inspiration and the base pressure (e.g., *PS = IPAP - EPAP*). In some contexts, pressure support means the difference which the ventilator aims to achieve, rather than what it actually achieves.

*Servo-ventilator*: A ventilator that measures patient ventilation, has a target ventilation, and which adjusts the level of pressure support to bring the patient ventilation towards the target ventilation.

*Spontaneous/Timed (S/T)*: A mode of a ventilator or other device that attempts to detect the initiation of a breath of a spontaneously breathing patient. If, however, the device is unable to detect a breath within a predetermined period of time, the device will automatically initiate delivery of the breath.

*Swing*: Equivalent term to pressure support.

*Triggered*: When a ventilator delivers a breath of *air* to a spontaneously breathing patient, it is said to be triggered to do so at the initiation of the respiratory portion of the breathing cycle by the patient's efforts.

### 6.2.4 Anatomy

### 6.2.4.1 Anatomy of the face

*Ala*: the external outer wall or "wing" of each nostril (plural: alar)

*Alare*: The most lateral point on the nasal *ala.*

*Alar curvature (or alar crest) point*: The most posterior point in the curved base line of each *ala,* found in the crease formed by the union of the *ala* with the cheek.

*Auricle*: The whole external visible part of the ear.

*(nose) Bony framework*: The bony framework of the nose comprises the nasal bones, the frontal process of the maxillae and the nasal part of the frontal bone.

*(nose) Cartilaginous framework*: The cartilaginous framework of the nose comprises the septal, lateral, major and minor cartilages.

*Columella*: the strip of skin that separates the nares and which runs from the pronasale to the upper lip.

*Columella angle*: The angle between the line drawn through the midpoint of the nostril aperture and a line drawn perpendicular to the Frankfort horizontal while intersecting subnasale.

*Frankfort horizontal plane*: A line extending from the most inferior point of the orbital margin to the left tragion. The tragion is the deepest point in the notch superior to the tragus of the auricle.

*Glabella*: Located on the soft tissue, the most prominent point in the midsagittal plane of the forehead.

*Lateral nasal cartilage*: A generally triangular plate of cartilage. Its superior margin is attached to the nasal bone and frontal process of the maxilla, and its inferior margin is connected to the greater alar cartilage.

*Greater alar cartilage*: A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils)*: Approximately ellipsoidal apertures forming the entrance to the nasal cavity. The singular form of nares is naris (nostril). The nares are separated by the nasal septum.

*Naso-labial sulcus or Naso-labial fold*: The skin fold or groove that runs from each side of the nose to the corners of the mouth, separating the cheeks from the upper lip.

*Naso-labial angle*: The angle between the columella and the upper lip, while intersecting subnasale.

*Otobasion inferior*: The lowest point of attachment of the auricle to the skin of the face.

*Otobasion superior*: The highest point of attachment of the auricle to the skin of the face.

*Pronasale*: the most protruded point or tip of the nose, which can be identified in lateral view of the rest of the portion of the head.

*Philtrum*: the midline groove that runs from lower border of the nasal septum to the top of the lip in the upper lip region.

*Pogonion*: Located on the soft tissue, the most anterior midpoint of the chin.

*Ridge (nasal)*: The nasal ridge is the midline prominence of the nose, extending from the Sellion to the Pronasale.

*Sagittal plane*: A vertical plane that passes from anterior (front) to posterior (rear). The midsagittal plane is a sagittal plane that divides the body into right and left halves.

*Sellion*: Located on the soft tissue, the most concave point overlying the area of the frontonasal suture.

*Septal cartilage (nasal)*: The nasal septal cartilage forms part of the septum and divides the front part of the nasal cavity.

*Subalare*: The point at the lower margin of the alar base, where the alar base joins with the skin of the superior (upper) lip.

*Subnasal point*: Located on the soft tissue, the point at which the columella merges with the upper lip in the midsagittal plane.

*Supramenton*: The point of greatest concavity in the midline of the lower lip between labrale inferius and soft tissue pogonion

### 6.2.4.2 Anatomy of the skull

*Frontal bone*: The frontal bone includes a large vertical portion, the *squama frontalis,* corresponding to the region known as the forehead.

*Mandible*: The mandible forms the lower jaw. The mental protuberance is the bony protuberance of the jaw that forms the chin.

*Maxilla*: The maxilla forms the upper jaw and is located above the mandible and below the orbits. The *frontal process of the maxilla* projects upwards by the side of the nose, and forms part of its lateral boundary.

*Nasal bones*: The nasal bones are two small oblong bones, varying in size and form in different individuals; they are placed side by side at the middle and upper part of the face, and form, by their junction, the "bridge" of the nose.

*Nasion*: The intersection of the frontal bone and the two nasal bones, a depressed area directly between the eyes and superior to the bridge of the nose.

*Occipital bone*: The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit*: The bony cavity in the skull to contain the eyeball.

*Parietal bones*: The parietal bones are the bones that, when joined together, form the roof and sides of the cranium.

*Temporal bones*: The temporal bones are situated on the bases and sides of the skull, and support that part of the face known as the temple.

*Zygomatic bones*: The face includes two zygomatic bones, located in the upper and lateral parts of the face and forming the prominence of the cheek.

### 6.2.4.3 Anatomy of the respiratory system

*Diaphragm*: A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx*: The larynx, or voice box houses the vocal folds and connects the inferior part of the pharynx (hypopharynx) with the trachea.

*Lungs*: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity*: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

**6.2.5 Patient interface**

*Anti-asphyxia valve (AAV):* The component or sub-assembly of a mask system that, by opening to atmosphere in a failsafe manner, reduces the risk of excessive $CO_2$ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame*: Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example, the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane*: Membrane will be taken to mean a typically thin element that has, preferably, substantially no resistance to bending, but has resistance to being stretched.

*Plenum chamber*: a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal*: May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell*: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Stiffener*: A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent*: (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

**6.2.6 Shape of structures**

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example, a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

To facilitate describing the shape of the three-dimensional structures and the surfaces, we first consider a cross-section through a surface of the structure at a point, *p*. An outward normal vector at *p* points away from the surface. In some examples we describe the surface from the point of view of an imaginary small person standing upright on the surface.

### 6.2.6.1 Curvature in one dimension

The curvature of a plane curve at p may be described as having a sign (e.g. positive, negative) and a magnitude (e.g. 1/radius of a circle that just touches the curve at *p*).

*Positive curvature:* If the curve at *p* turns towards the outward normal, the curvature at that point will be taken to be positive (if the imaginary small person leaves the point *p* they must walk uphill). Such curves are often referred to as concave.

*Zero curvature:* If the curve at *p* is a straight line, the curvature will be taken to be zero (if the imaginary small person leaves the point *p*, they can walk on a level, neither up nor down).

*Negative curvature:* If the curve at *p* turns away from the outward normal, the curvature in that direction at that point will be taken to be negative (if the imaginary small person leaves the point *p* they must walk downhill). Such curves are often referred to as convex.

### 6.2.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small.

*Principal curvatures and directions:* The directions of the normal planes where the curvature of the curve takes its maximum and minimum values are called the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from f(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 6.2.6.3 Space curves

*Space curves*: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix. A typical human right ear comprises a helix, which is a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule, or alternatively by a left-hand rule.

*Osculating plane:* The plane containing the unit tangent vector and the unit principal normal vector..

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path).

A space curve turning towards the direction of the right-hand binormal may be considered as having a right-hand positive torsion (e.g. a right-hand helix). A space curve turning away from the direction of the right-hand binormal may be considered as having a right-hand negative torsion (e.g. a left-hand helix).

Equivalently, and with reference to a left-hand rule, a space curve turning towards the direction of the left-hand binormal may be considered as having a left-hand positive torsion (e.g. a left-hand helix). Hence left-hand positive is equivalent to right-hand negative.

### 6.2.6.4 Holes

A surface may have a one-dimensional hole, e.g. a hole bounded by a plane curve or by a space curve. Thin structures (e.g. a membrane) with a hole, may be described as having a one-dimensional hole.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. In a yet another example, a conduit may comprise a one-dimension hole (e.g. at its entrance or at its exit), and a two-dimension hole bounded by the inside surface of the conduit.

### 6.3 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

6.4 REFERENCE SIGNS LIST

| | |
|---|---|
| patient | 1000 |
| bed partner | 1100 |
| patient interface | 3000 |
| seal-forming structure | 3100 |
| plenum chamber | 3200 |
| chord | 3210 |
| superior point | 3220 |
| positioning and stabilising structure | 3300 |
| vent | 3400 |
| connection port | 3600 |
| forehead support | 3700 |
| patient interface | 3800 |
| nasal prong | 3810 |
| air supply lumens | 3820 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| air filter | 4110 |

(continued)

| | |
|---|---|
| inlet air filter | 4112 |
| outlet air filter | 4114 |
| muffler | 4120 |
| inlet muffler | 4122 |
| outlet muffler | 4124 |
| outlet | 4130 |
| pressure generator | 4140 |
| blower | 4142 |
| motor | 4144 |
| anti - spill back valve | 4160 |
| air circuit | 4170 |
| supplemental oxygen | 4180 |
| electrical components | 4200 |
| Printed Circuit Board Assembly (PCBA) | 4202 |
| electrical power supply | 4210 |
| input devices | 4220 |
| central controller | 4230 |
| clock | 4232 |
| therapy device controller | 4240 |
| protection circuits | 4250 |
| memory | 4260 |
| transducers | 4270 |
| pressure sensor | 4272 |
| flow rate sensor | 4274 |
| motor speed transducer | 4276 |
| data communication interface | 4280 |
| remote external communication network | 4282 |
| local external communication network | 4284 |
| remote external device | 4286 |
| local external device | 4288 |
| output devices | 4290 |
| display driver | 4292 |
| display | 4294 |
| humidifier connector | 4296 |
| algorithms | 4300 |
| pre-processing module | 4310 |
| interface pressure estimation algorithm | 4312 |
| vent flow rate estimation | 4314 |
| leak flow rate estimation | 4316 |
| respiratory flow rate estimation | 4318 |

(continued)

| | |
|---|---|
| therapy engine module | 4320 |
| phase determination algorithm | 4321 |
| waveform determination algorithm | 4322 |
| ventilation determination algorithm | 4323 |
| inspiratory flow limitation determination algorithm | 4324 |
| apnea / hypopnea determination algorithm | 4325 |
| snore determination algorithm | 4326 |
| airway patency determination algorithm | 4327 |
| target ventilation determination algorithm | 4328 |
| therapy parameter determination algorithm | 4329 |
| therapy control module | 4330 |
| methods | 4340 |
| humidifier | 5000 |
| humidifier inlet | 5002 |
| humidifier outlet | 5004 |
| humidifier base | 5006 |
| reservoir | 5110 |
| conductive portion | 5120 |
| humidifier reservoir dock | 5130 |
| locking lever | 5135 |
| water level indicator | 5150 |
| humidifier transducer | 5210 |
| air pressure sensor | 5212 |
| air flow rate transducer | 5214 |
| temperature sensor | 5216 |
| heating element | 5240 |
| humidifier controller | 5250 |
| central humidifier controller | 5251 |
| heating element controller | 5252 |
| air circuit controller | 5254 |
| external housing | 5300 |
| upper portion | 5302 |
| lower portion | 5304 |
| lid | 5306 |
| chassis | 5310 |
| vertical chassis wall | 5312 |
| chamber inlet port | 5314 |
| chassis peripheral wall | 5316 |
| free end | 5318 |
| inner chassis wall portion | 5320 |

| | |
|---|---|
| superior facing surface | 5322 |
| end cap | 5330 |
| main cap wall | 5332 |
| air inlet port | 5334 |
| inner surface | 5336 |
| external annular port wall | 5338 |
| inner peripheral wall | 5340 |
| free end | 5342 |
| locating feature | 5344 |
| resilient seal | 5360 |
| liquid trap portion | 5362 |
| first lip | 5364 |
| air inlet port portion | 5366 |
| second lip | 5368 |
| flange portion | 5370 |
| peripheral seal portion | 5372 |
| seal runner portion | 5374 |
| liquid trap | 5380 |
| vertical separation | 5382 |
| superior portion | 5384 |
| lateral portion | 5386 |
| inverted receptacle | 5600 |
| closed end | 5602 |
| open end | 5604 |
| receptacle wall | 5606 |
| drain hole | 5608 |
| interior trap wall | 5700 |
| baffle portion | 5800 |
| baffle extension | 5802 |
| radially extending baffle portion | 5804 |
| baffle member | 5806 |
| baffle legs | 5808 |
| liquid trap partition | 5850 |
| first portion | 5852 |
| second portion | 5854 |
| upright intermediary wall | 5856 |
| dividing wall | 5858 |
| partition wall | 5860 |
| first gap | 5862 |
| second gap | 5864 |

## EP 4 081 285 B1

(continued)

| first liquid trap catchment | 5870 |
|---|---|
| second liquid trap catchment | 5872 |
| third liquid trap catchment | 5874 |
| moveable barrier | 5900 |
| barrier body | 5902 |
| pivot | 5904 |
| stored position | 5910 |
| closed position | 5912 |
| secondary closure element | 5950 |

## Claims

1. An apparatus (5000) to change the absolute humidity of a flow of air for delivery to an entrance of the airways of a patient, the change being compared to the absolute humidity of ambient air, the apparatus comprising:

   a reservoir (5110) configured to hold a volume of liquid;
   a heating element (5240) to create vapour from the liquid;
   a chamber to mix the flow of air with the vapour;
   a body (5310), the body comprising a first wall structure (5312) comprising a chamber inlet port (5314), wherein the first wall structure (5312) is generally upright when the apparatus (5000) is in an intended working orientation;
   a closure element (5330) comprising an air inlet port (5334) for pneumatically connecting to a source (4000) of the flow of air;
   wherein the closure element (5330) is secured to the body (5310) to provide a sealed gas flow path between the air inlet port (5334) and the chamber inlet port (5314), and a liquid trap (5380) in the gas flow path, **characterized in that** the chamber inlet port (5314) is positioned relative to the air inlet port (5334) such that when the humidifier (5110) is in an intended working orientation, a lowest point of the chamber inlet port (5314) is inferior to a lowest point of the air inlet port (5334).

2. The apparatus (5000) of claim 1, wherein the first wall structure (5312) comprises a chamber inlet port sealing portion surrounding the chamber inlet port (5314) and extending towards the closure element (5330), optionally wherein the chamber inlet port sealing portion comprises a first peripheral wall (5316).

3. The apparatus (5000) of any one of claims 1 to 2, wherein the closure element (5330) comprises an air inlet port sealing portion (5340) surrounding the air inlet port (5334) and extending towards the first wall structure (5312).

4. The apparatus (5000) of claim 2, comprising a resilient seal (5360), wherein the closure element (5330) comprises an air inlet port sealing portion (5340) surrounding the air inlet port (5334) and extending towards the first wall structure (5312), and wherein the resilient seal (5360) comprises any of:

   a liquid trap portion (5362) positioned between the chamber inlet port sealing portion (5316) and the air inlet port sealing portion (5340),
   a liquid trap portion (5362) provided on a rim of the air inlet port sealing portion (5340) and configured to seal against the chamber inlet port sealing portion (5316).

5. The apparatus (5000) of any one of claims 1 to 4, wherein the closure element (5330) is generally upright when the apparatus (5000) is in the intended working orientation.

6. The apparatus (5000) of any one of claims 1 to 5, wherein when the apparatus is in an intended working orientation, a lowest point of the chamber inlet port (5314) is one or more of: inferior to a lowest point of the air inlet port (5334), and superior to an intended liquid fill level of the chamber.

7. The apparatus (5000) of any one of claims 1 to 6, wherein a centre of the chamber inlet port (5314) is at least one of:

48

laterally offset from a centre of the air inlet port (5334), and inferior to a centre of the air inlet port (5334).

8. The apparatus (5000) of any one of claims 1 to 7, wherein the liquid trap (5380) is configured to trap a volume of liquid of one of: between about 5 ml to about 100 ml, between about 10 ml to about 80 ml, between about 30 ml to about 80 ml, and about 60 ml.

9. The apparatus of any one of claims 1 to 8, wherein the liquid trap (5380) comprises a superior portion (5384) provided above the chamber, optionally wherein the superior portion (5384) of the liquid trap (5380) is configured such that liquid drains from the superior portion (5384) into an inferior portion of the liquid trap (5380), and/or wherein the liquid trap (5380) comprises a lateral portion (5386),

optionally wherein a height of the lateral portion (5386) is less than a greatest height of the liquid trap and/or the lateral portion (5386) is configured such that liquid drains from the lateral portion (5386) into an inferior portion of the liquid trap (5380).

10. The apparatus (5000) of any one of claims 1 to 9, comprising an inverted receptacle (5600) provided to a superior portion of the chamber inlet port (5314) when the apparatus (5000) is in an intended working orientation, optionally wherein the inverted receptacle (5600) extends from the body towards the closure element (5330).

11. The apparatus (5000) of any one of claims 1 to 10, comprising an interior trap wall (5700) extending from the closure element (5330) towards the chamber inlet port (5314) and surrounding the air inlet port (5334).

12. The apparatus (5000) of any one of claims 1 to 11, comprising a baffle portion (5800) positioned between the air inlet port (5334) and the chamber inlet port (5314), optionally wherein the baffle portion (5800) is configured to direct liquid flowing from the chamber inlet port (5314) in a radial direction.

13. The apparatus (5000) of any one of claims 1 to 12, comprising at least one liquid trap partition (5850) providing a plurality of liquid trap catchments within the liquid trap (5380), optionally wherein the liquid trap comprises a first liquid trap catchment inferior to the chamber inlet port (5314), and a second liquid trap catchment inferior to the first liquid trap catchment when the apparatus (5000) is in an inverted from intended working orientation.

14. The apparatus of claim 13, wherein the at least one liquid trap partition (5850) comprises an upright intermediary wall and a partition wall (5860) extending from the upright intermediary wall towards the body, wherein a superior gap is provided between the chamber inlet port (5314) and the partition wall (5860) and the superior gap opens into the first liquid trap catchment; and/or
the at least one liquid trap partition (5850) comprises an interior trap wall (5854) extending from the closure element (5330) towards the chamber inlet port (5314) and surrounding the air inlet port (5334) to define a third liquid trap catchment around the interior trap wall.

15. The apparatus (5000) of any one of claims 1 to 14, wherein the closure element (5330) is configured to interact with a secondary closure element (5950) to provide the liquid trap, optionally wherein the secondary closure element (5950) comprises at least the air inlet port for pneumatically connecting to a source of the flow of air, wherein, optionally, the secondary closure element (5950) is a component of a device configured to provide the source (4000) of the flow of air, preferably a component of a Positive Airway Pressure generator configured to be connected to the apparatus (5000), or, optionally, wherein the secondary closure element (5950) is configured to act as an interface between the apparatus (5000) and a device configured to provide the source of the flow of air.

16. The apparatus of any one of claims 1 to 15, further comprising:

a source (4000) of a flow of air at positive pressure to ambient;
a body (5300) constructed and arranged to be fixed in location in use relative to the source (4000);
an inlet pneumatic connection structure (5002) for connecting to the source (4000) to receive sealably the flow of air at positive pressure from the source in use;
a temperature sensor (5216);
a controller (5250) to control the heating element (5240); and
an outlet pneumatic connection structure (5004) to receive the flow of air with increased absolute humidity;
wherein the reservoir (5110) is configured to direct the flow of air so that the flow of air contacts a surface of the volume of liquid in use so that water vapour may transfer from the volume of liquid to the flow of air in use to increase the absolute humidity of the flow of air, the reservoir (5110) including a wall constructed at least in part from a material having a relatively high thermal conductivity,

wherein the body (5300) is configured to hold the reservoir (5110) in location close relative to the heating element (5240) so that heat energy may transfer from the heating element (5240) to the volume of liquid to increase the absolute humidity of the flow of air,

wherein the controller (5250) is constructed and arranged to energise the heating element to heat the liquid without boiling the liquid,

wherein the apparatus includes a sealing arrangement so that in use the flow of air with increased absolute humidity received at the outlet pneumatic connection structure (5004) has a positive pressure with respect to ambient,

wherein the inlet pneumatic connection structure (5002) comprises the air inlet port (5334).

17. A device for treating a respiratory disorder comprising:

a Positive Airway Pressure generator (4000); and

an apparatus (5000) to change the absolute humidity of a flow of air produced by the Positive Airway Pressure generator (4000) for delivery to an entrance of the airways of a patient, wherein the apparatus is configured as claimed in any one of claims 1 to 15.

**Patentansprüche**

1. Vorrichtung (5000) zur Änderung der absoluten Feuchtigkeit einer Luftströmung zur Abgabe zu einem Eingang der Luftwege eines Patienten, wobei die Änderung mit der absoluten Feuchtigkeit von Umgebungsluft verglichen wird, wobei die Vorrichtung aufweist;

einen Behälter (5110), der so konfiguriert ist, dass er ein Flüssigkeitsvolumen enthält; ein Heizelement (5240), um Dampf aus der Flüssigkeit zu erzeugen;

eine Kammer, um die Luftströmung mit dem Dampf zu mischen;

einen Körper (5310), wobei der Körper eine erste Wandstruktur (5312) mit einem Kammereinlassanschluss (5314) aufweist, wobei die erste Wandstruktur (5312) allgemein aufrecht steht, wenn sich die Vorrichtung (5000) in einer beabsichtigten Arbeitsorientierung befindet;

ein Verschlusselement (5330) aufweisend einen Lufteinlassanschluss (5334) zum pneumatischen Verbinden mit einer Quelle (4000) der Luftströmung;

wobei das Verschlusselement (5330) am Körper (5310) befestigt ist, um einen abgedichteten Gasströmungsweg zwischen dem Lufteinlassanschluss (5334) und dem Kammereinlassanschluss (5314) und eine Flüssigkeitsfalle (5380) im Gasströmungsweg vorzusehen,

**dadurch gekennzeichnet, dass** der Kammereinlassanschluss (5314) relativ zum Lufteinlassanschluss (5334) so positioniert ist, dass in einer beabsichtigten Arbeitsorientierung des Befeuchters (5110) ein tiefster Punkt des Kammereinlassanschlusses (5314) unter einem tiefsten Punkt des Lufteinlassanschlusses (5334) liegt.

2. Vorrichtung (5000) nach Anspruch 1, wobei die erste Wandstruktur (5312) einen Kammereinlassanschluss-Dichtungsabschnitt aufweist, der den Kammereinlassanschluss (5314) umgibt und sich hin zum Verschlusselement (5330) erstreckt, wobei optional der Kammereinlassanschluss-Dichtungsabschnitt eine erste Umfangswand (5316) aufweist.

3. Vorrichtung (5000) nach einem der Ansprüche 1 bis 2, wobei das Verschlusselement (5330) einen Lufteinlassanschluss-Dichtungsabschnitt (5340) aufweist, der den Lufteinlassanschluss (5334) umgibt und sich hin zur ersten Wandstruktur (5312) erstreckt.

4. Vorrichtung (5000) nach Anspruch 2 mit einer elastischen Dichtung (5360), wobei das Verschlusselement (5330) einen Lufteinlassanschluss-Dichtungsabschnitt (5340) aufweist, der den Lufteinlassanschluss (5334) umgibt und sich hin zur ersten Wandstruktur (5312) erstreckt, und wobei die elastische Dichtung (5360) aufweist:

einen Flüssigkeitsfallenabschnitt (5362), der zwischen dem Kammereinlassanschluss-Dichtungsabschnitt (5316) und dem Lufteinlassanschluss-Dichtungsabschnitt (5340) positioniert ist, oder

einen Flüssigkeitsfallenabschnitt (5362), der auf einem Rand des Kammereinlassanschluss-Dichtungsabschnitts (5316) vorgesehen und so konfiguriert ist, dass er am Kammereinlassanschluss-Dichtungsabschnitt (5316) abdichtet.

5. Vorrichtung (5000) nach einem der Ansprüche 1 bis 4, wobei das Verschlusselement (5330) allgemein aufrecht steht, wenn sich die Vorrichtung (5000) in der beabsichtigten Arbeitsorientierung befindet.

6. Vorrichtung (5000) nach einem der Ansprüche 1 bis 5, wobei in einer beabsichtigten Arbeitsorientierung der Vorrichtung ein tiefster Punkt des Kammereinlassanschlusses (5314) unter einem tiefsten Punkt des Lufteinlassanschlusses (5334) und/oder über einem beabsichtigten Flüssigkeitsfüllstand der Kammer liegt.

7. Vorrichtung (5000) nach einem der Ansprüche 1 bis 6, wobei eine Mitte des Kammereinlassanschlusses (5314) seitlich versetzt von einer Mitte des Lufteinlassanschlusses (5334) und/oder unter einer Mitte des Lufteinlassanschlusses (5334) liegt.

8. Vorrichtung (5000) nach einem der Ansprüche 1 bis 7, wobei die Flüssigkeitsfalle (5380) so konfiguriert ist, dass sie ein Flüssigkeitsvolumen zwischen etwa 5 ml bis etwa 100 ml, zwischen etwa 10 ml bis etwa 80 ml, zwischen etwa 30 ml bis etwa 80 ml oder von etwa 60 ml auffängt.

9. Vorrichtung nach einem der Ansprüche 1 bis 8, wobei die Flüssigkeitsfalle (5380) einen oberen Abschnitt (5384) aufweist, der über der Kammer vorgesehen ist, wobei optional der obere Abschnitt (5384) der Flüssigkeitsfalle (5380) so konfiguriert ist, dass Flüssigkeit aus dem oberen Abschnitt (5384) in einen unteren Abschnitt der Flüssigkeitsfalle (5380) abläuft, und/oder wobei die Flüssigkeitsfalle (5380) einen Seitenabschnitt (5386) aufweist, wobei optional eine Höhe des Seitenabschnitts (5386) kleiner ist als eine größte Höhe der Flüssigkeitsfalle und/oder der Seitenabschnitt (5386) so konfiguriert ist, dass Flüssigkeit aus dem Seitenabschnitt (5386) in einen unteren Abschnitt der Flüssigkeitsfalle (5380) abläuft.

10. Vorrichtung (5000) nach einem der Ansprüche 1 bis 9 aufweisend ein umgekehrtes Behältnis (5600), das an einem oberen Abschnitt des Kammereinlassanschlusses (5314) vorgesehen ist, wenn sich die Vorrichtung (5000) in einer beabsichtigten Arbeitsorientierung befindet, wobei sich optional das umgekehrte Behältnis (5600) vom Körper hin zum Verschlusselement (5330) erstreckt.

11. Vorrichtung (5000) nach einem der Ansprüche 1 bis 10 mit einer inneren Fallenwand (5700), die sich vom Verschlusselement (5330) hin zum Kammereinlassanschluss (5314) erstreckt und den Lufteinlassanschluss (5334) umgibt.

12. Vorrichtung (5000) nach einem der Ansprüche 1 bis 11 mit einem Ablenkabschnitt (5800), der zwischen dem Lufteinlassanschluss (5334) und dem Kammereinlassanschluss (5314) positioniert ist, wobei optional der Ablenkabschnitt (5800) so konfiguriert ist, dass er aus dem Kammereinlassanschluss (5314) strömende Flüssigkeit in eine Radialrichtung lenkt.

13. Vorrichtung (5000) nach einem der Ansprüche 1 bis 12 aufweisend mindestens eine Flüssigkeitsfallenabtrennung (5850), die mehrere Flüssigkeitsfallensammler in der Flüssigkeitsfalle (5380) bereitstellt, wobei optional die Flüssigkeitsfalle einen ersten Flüssigkeitsfallensammler unter dem Kammereinlassanschluss (5314) und einen zweiten Flüssigkeitsfallensammler unter dem ersten Flüssigkeitsfallensammler aufweist, wenn die Vorrichtung (5000) gegenüber der beabsichtigten Arbeitsorientierung umgekehrt steht.

14. Vorrichtung nach Anspruch 13, wobei die mindestens eine Flüssigkeitsfallenabtrennung (5850) eine aufrechte Zwischenwand und eine sich von der aufrechten Zwischenwand hin zum Körper erstreckende Trennwand (5860) aufweist, wobei ein oberer Spalt zwischen dem Kammereinlassanschluss (5314) und der Trennwand (5860) vorgesehen ist und der obere Spalt im ersten Flüssigkeitsfallensammler mündet; und/oder die mindestens eine Flüssigkeitsfallenabtrennung (5850) eine innere Fallenwand (5854) aufweist, die sich vom Verschlusselement (5330) hin zum Kammereinlassanschluss (5314) erstreckt und den Lufteinlassanschluss (5334) umgibt, um einen dritten Flüssigkeitsfallensammler um die innere Fallenwand herum zu bilden.

15. Vorrichtung (5000) nach einem der Ansprüche 1 bis 14, wobei das Verschlusselement (5330) so konfiguriert ist, dass es mit einem sekundären Verschlusselement (5950) in Wechselwirkung steht, um die Flüssigkeitsfalle bereitzustellen, wobei optional das sekundäre Verschlusselement (5950) mindestens den Lufteinlassanschluss zum pneumatischen Verbinden mit einer Quelle der Luftströmung aufweist, wobei optional das sekundäre Verschlusselement (5950) eine Komponente eines Geräts ist, das so konfiguriert ist, dass es die Quelle (4000) der Luftströmung

# EP 4 081 285 B1

bereitstellt, vorzugsweise eine Komponente eines Generators für positiven Atemwegdruck, der so konfiguriert ist, dass er mit der Vorrichtung (5000) verbunden ist, oder wobei optional das sekundäre Verschlusselement (5950) so konfiguriert ist, dass es als Schnittstelle zwischen der Vorrichtung (5000) einem Gerät wirkt, das so konfiguriert ist, dass es die Quelle der Luftströmung bereitstellt.

16. Vorrichtung nach einem der Ansprüche 1 bis 15, die ferner aufweist:

eine Quelle (4000) einer Luftströmung mit positivem Druck gegenüber der Umgebung;
einen Körper (5300), der so aufgebaut und angeordnet ist, dass er im Gebrauch relativ zur Quelle (4000) an Ort und Stelle feststehend ist;
eine pneumatische Einlassverbindungsstruktur (5002) zum Verbinden mit der Quelle (4000), um die Luftströmung mit positivem Druck von der Quelle im Gebrauch abgedichtet zu empfangen;
einen Temperatursensor (5216);
eine Steuerung (5250), um das Heizelement (5240) zu steuern; und
eine pneumatische Auslassverbindungsstruktur (5004), um die Luftströmung mit erhöhter absoluter Feuchtigkeit zu empfangen;
wobei der Behälter (5110) so konfiguriert ist, dass er die Luftströmung lenkt, so dass die Luftströmung eine Oberfläche des Flüssigkeitsvolumens im Gebrauch kontaktiert, so dass Wasserdampf vom Flüssigkeitsvolumen zur Luftströmung im Gebrauch übergehen kann, um die absolute Feuchtigkeit der Luftströmung zu erhöhen,
wobei der Behälter (5110) eine Wand aufweist, die zumindest teilweise aus einem Material mit einer relativ hohen Wärmeleitfähigkeit aufgebaut ist,
wobei der Körper (5300) so konfiguriert ist, dass er den Behälter (5110) an Ort und Stelle nahe am Heizelement (5240) hält, so dass Wärmeenergie vom Heizelement (5240) zum Flüssigkeitsvolumen übergehen kann, um die absolute Feuchtigkeit der Luftströmung zu erhöhen,
wobei die Steuerung (5250) so aufgebaut und angeordnet ist, dass sie das Heizelement speist, um die Flüssigkeit ohne Sieden der Flüssigkeit zu beheizen,
wobei die Vorrichtung eine Dichtungsanordnung aufweist, so dass im Gebrauch die Luftströmung mit erhöhter absoluter Feuchtigkeit, die an der pneumatischen Auslassverbindungsstruktur (5004) empfangen wird, einen positiven Druck im Hinblick auf die Umgebung hat,
wobei die pneumatische Einlassverbindungsstruktur (5002) den Lufteinlassanschluss (5334) aufweist.

17. Gerät zur Behandlung einer Atemstörung, das aufweist:

einen Generator (4000) für positiven Atemwegdruck; und
eine Vorrichtung (5000) zur Änderung der absoluten Feuchtigkeit einer durch den Generator (4000) für positiven Atemwegdruck erzeugten Luftströmung zur Abgabe zu einem Eingang der Luftwege eines Patienten, wobei die Vorrichtung nach einem der Ansprüche 1 bis 15 konfiguriert ist.

## Revendications

1. Appareil (5000) pour modifier l'humidité absolue d'un flux d'air à des fins d'administration à une entrée des voies aériennes d'un patient, la modification étant comparée à l'humidité absolue d'air ambiant, l'appareil comprenant :

un réservoir (5110) configuré pour contenir un volume de liquide ;
un élément chauffant (5240) pour créer de la vapeur à partir du liquide ;
une chambre pour mélanger le flux d'air à la vapeur ;
un corps (5310), le corps comprenant une première structure de paroi (5312) comprenant un orifice d'entrée de chambre (5314), dans lequel la première structure de paroi (5312) est globalement verticale lorsque l'appareil (5000) est dans une orientation de travail voulue ;
un élément de fermeture (5330) comprenant un orifice d'entrée d'air (5334) destiné à un raccordement pneumatique à une source (4000) du flux d'air ;
dans lequel l'élément de fermeture (5330) est fixé au corps (5310) pour fournir un trajet d'écoulement de gaz étanche entre l'orifice d'entrée d'air (5334) et l'orifice d'entrée de chambre (5314), et un piège à liquide (5380) dans le trajet d'écoulement de gaz,
caractérisé en ce que l'orifice d'entrée de chambre (5314) est positionné par rapport à l'orifice d'entrée d'air (5334) de sorte que, lorsque l'humidificateur (5110) est dans une orientation de travail voulue, un point le plus bas de l'orifice d'entrée de chambre (5314) est plus bas qu'un point le plus bas de l'orifice d'entrée d'air (5334).

**2.** Appareil (5000) selon la revendication 1, dans lequel la première structure de paroi (5312) comprend une partie d'étanchéité d'orifice d'entrée de chambre entourant l'orifice d'entrée de chambre (5314) et s'étendant vers l'élément de fermeture (5330), éventuellement dans lequel la partie d'étanchéité d'orifice d'entrée de chambre comprend une première paroi périphérique (5316).

**3.** Appareil (5000) selon l'une ou l'autre des revendications 1 et 2, dans lequel l'élément de fermeture (5330) comprend une partie d'étanchéité d'orifice d'entrée d'air (5340) entourant l'orifice d'entrée d'air (5334) et s'étendant vers la première structure de paroi (5312).

**4.** Appareil (5000) selon la revendication 2, comprenant un joint d'étanchéité élastique (5360), dans lequel l'élément de fermeture (5330) comprend une partie d'étanchéité d'orifice d'entrée d'air (5340) entourant l'orifice d'entrée d'air (5334) et s'étendant vers la première structure de paroi (5312), et dans lequel le joint d'étanchéité élastique (5360) comprend une quelconque partie parmi :

une partie piège à liquide (5362) positionnée entre la partie d'étanchéité d'orifice d'entrée de chambre (5316) et la partie d'étanchéité d'orifice d'entrée d'air (5340),
une partie piège à liquide (5362) disposée sur un rebord de la partie d'étanchéité d'orifice d'entrée d'air (5340) et configurée pour assurer une étanchéité contre la partie d'étanchéité d'orifice d'entrée de chambre (5316).

**5.** Appareil (5000) selon l'une quelconque des revendications 1 à 4, dans lequel l'élément de fermeture (5330) est globalement vertical lorsque l'appareil (5000) est dans l'orientation de travail voulue.

**6.** Appareil (5000) selon l'une quelconque des revendications 1 à 5, dans lequel, lorsque l'appareil est dans une orientation de travail voulue, un point le plus bas de l'orifice d'entrée de chambre (5314) est : plus bas qu'un point le plus bas de l'orifice d'entrée d'air (5334) et/ou plus haut qu'un niveau de remplissage à liquide voulu de la chambre.

**7.** Appareil (5000) selon l'une quelconque des revendications 1 à 6, dans lequel un centre de l'orifice d'entrée de chambre (5314) est : décalé latéralement d'un centre de l'orifice d'entrée d'air (5334) et/ou plus bas qu'un centre de l'orifice d'entrée d'air (5334).

**8.** Appareil (5000) selon l'une quelconque des revendications 1 à 7, dans lequel le piège à liquide (5380) est configuré pour piéger un volume de liquide qui est l'un parmi :

un volume compris entre environ 5 ml et environ 100 ml,
un volume compris entre environ 10 ml et environ 80 ml,
un volume compris entre environ 30 ml et environ 80 ml, et
un volume d'environ 60 ml.

**9.** Appareil selon l'une quelconque des revendications 1 à 8, dans lequel le piège à liquide (5380) comprend une partie supérieure (5384) disposée au-dessus de la chambre, avec éventuellement la partie supérieure (5384) du piège à liquide (5380) configurée de sorte qu'un liquide s'évacue de la partie supérieure (5384) dans une partie inférieure du piège à liquide (5380),
et/ou dans lequel le piège à liquide (5380) comprend une partie latérale (5386), avec éventuellement une hauteur de la partie latérale (5386) inférieure à une hauteur la plus grande du piège à liquide et/ou la partie latérale (5386) est configurée de sorte qu'un liquide s'évacue de la partie latérale (5386) dans une partie inférieure du piège à liquide (5380).

**10.** Appareil (5000) selon l'une quelconque des revendications 1 à 9, comprenant un réceptacle inversé (5600) disposé sur une partie supérieure de l'orifice d'entrée de chambre (5314) lorsque l'appareil (5000) est dans une orientation de travail voulue, éventuellement dans lequel le réceptacle inversé (5600) s'étend du corps vers l'élément de fermeture (5330).

**11.** Appareil (5000) selon l'une quelconque des revendications 1 à 10, comprenant une paroi de piège intérieure (5700) s'étendant de l'élément de fermeture (5330) vers l'orifice d'entrée de chambre (5314) et entourant l'orifice d'entrée d'air (5334).

**12.** Appareil (5000) selon l'une quelconque des revendications 1 à 11, comprenant une partie chicane (5800) positionnée entre l'orifice d'entrée d'air (5334) et l'orifice d'entrée de chambre (5314), optionnellement avec la partie chicane (5800) configurée pour diriger un liquide s'écoulant de l'orifice d'entrée de chambre (5314) dans une direction radiale.

13. Appareil (5000) selon l'une quelconque des revendications 1 à 12, comprenant au moins une cloison de piège à liquide (5850) fournissant une pluralité de captages à l'intérieur du piège à liquide (5380), éventuellement dans lequel le piège à liquide comprend un premier captage de piège à liquide plus bas que l'orifice d'entrée de chambre (5314), et un deuxième captage de piège à liquide plus bas que le premier captage de piège à liquide lorsque l'appareil (5000) est dans une orientation inversée par rapport à l'orientation de travail voulue.

14. Appareil selon la revendication 13, dans lequel l'au moins une cloison de piège à liquide (5850) comprend une paroi intermédiaire verticale et une paroi de cloison (5860) s'étendant de la paroi intermédiaire verticale vers le corps, dans lequel un espace supérieur est prévu entre l'orifice d'entrée de chambre (5314) et la paroi de cloison (5860) et l'espace supérieur débouche dans le premier captage de piège à liquide ; et/ou
l'au moins une cloison de piège à liquide (5850) comprend une paroi de piège intérieure (5854) s'étendant de l'élément de fermeture (5330) vers l'orifice d'entrée de chambre (5314) et entourant l'orifice d'entrée d'air (5334) pour délimiter un troisième captage de piège à liquide autour de la paroi de piège intérieure.

15. Appareil (5000) selon l'une quelconque des revendications 1 à 14, dans lequel l'élément de fermeture (5330) est configuré pour interagir avec un élément de fermeture secondaire (5950) de façon à obtenir le piège à liquide, éventuellement dans lequel l'élément de fermeture secondaire (5950) comprend au moins l'orifice d'entrée d'air destiné à un raccordement pneumatique à une source du flux d'air, dans lequel, éventuellement, l'élément de fermeture secondaire (5950) est un composant d'un dispositif configuré pour fournir la source (4000) du flux d'air, de préférence un composant d'un générateur de pression positive dans les voies respiratoires configuré pour être raccordé à l'appareil (5000), ou, éventuellement, dans lequel l'élément de fermeture secondaire (5950) est configuré pour servir d'interface entre l'appareil (5000) et un dispositif configuré pour fournir la source du flux d'air.

16. Appareil selon l'une quelconque des revendications 1 à 15, comprenant en outre :

une source (4000) d'un flux d'air à pression positive par rapport à la pression ambiante ;
un corps (5300) structuré et conçu pour être, en utilisation, fixé en position par rapport à la source (4000) ;
une structure de raccordement pneumatique d'entrée (5002) destinée à être raccordée à la source (4000) pour recevoir de manière étanche le flux d'air à pression positive de la source en utilisation ;
un capteur de température (5216) ;
un organe de commande (5250) pour commander l'élément chauffant (5240) ; et
une structure de raccordement pneumatique de sortie (5004) pour recevoir le flux d'air ayant une humidité absolue augmentée ;
dans lequel le réservoir (5110) est configuré pour diriger le flux d'air de sorte que le flux d'air contacte une surface du volume de liquide en utilisation pour que de la vapeur d'eau puisse être transférée du volume de liquide au flux d'air en utilisation pour augmenter l'humidité absolue du flux d'air, le réservoir (5110) comprenant une paroi structurée au moins en partie à base d'un matériau ayant une conductivité thermique relativement élevée,
dans lequel le corps (5300) est configuré pour maintenir le réservoir (5110) en position proche de l'élément chauffant (5240) de sorte que de l'énergie thermique puisse être transférée de l'élément chauffant (5240) vers le volume de liquide pour augmenter l'humidité absolue du flux d'air,
dans lequel l'organe de commande (5250) est structuré et conçu pour alimenter en énergie l'élément chauffant pour chauffer le liquide sans faire bouillir le liquide,
dans lequel l'appareil comprend un agencement d'étanchéité de sorte que, en utilisation, le flux d'air ayant une humidité absolue augmentée reçu au niveau de la structure de raccordement pneumatique de sortie (5004) ait une pression positive par rapport à la pression ambiante,
dans lequel la structure de raccordement pneumatique d'entrée (5002) comprend l'orifice d'entrée d'air (5334).

17. Dispositif de traitement d'un trouble respiratoire, comprenant :

un générateur de pression positive dans les voies respiratoires (4000) ; et
un appareil (5000) pour modifier l'humidité absolue d'un flux d'air produit par le générateur de pression positive dans les voies respiratoires (4000) à des fins d'administration à une entrée des voies aériennes d'un patient, dans lequel l'appareil est configuré selon l'une quelconque des revendications 1 à 15.

FIG. 1A

FIG. 1B

FIG. 1C

EP 4 081 285 B1

**FIG. 2A**

Copyright 2012 ResMed Limited

Nasal cavity

Nasal bone

Lateral nasal cartilage

Greater alar cartilage

Nostril

Lip superior

Lip inferior

Hard palate

Soft palate

Oropharynx

Tongue

Epiglottis

Vocal folds

Larynx

Esophagus

Trachea

# FIG. 2B

Copyright 2012 ResMed Limited

**FIG. 3A**

**FIG. 3B**

4015

4000

4018

4112

4220

4015

4012

Superior

Posterior

Anterior

Inferior

4202

4142

4100, 4020

4200

4010

4016

4210

4014

**FIG. 4A**

4110 — 4112 Air inlet filter ← Supplementary O₂

Pneumatic block

4120 — 4122 Inlet muffler

4180

4270 — Transducer(s)

4140 — 4142 Blower | 4144 Motor

4020

4120 — 4124 Outlet muffler

Upstream

4270 — Transducer(s)

↕

Downstream

4160 — Anti-spillback valve

5000 — Humidifier

4110 — 4114 Filter

4180

4170 — Air circuit / delivery tube ← Supplementary O₂

4270 — Transducer(s)

4180

**FIG. 4B**

3000 — Patient Interface ← Supplementary O₂

**FIG. 4C**

EP 4 081 285 B1

**FIG. 4C**

4300

4310

Preprocessing

| Interface pressure estimation | Vent flow rate estimation | | Leak flow rate estimation |

4312    4314    4316

Respiratory flow rate estimation

4318

4320

4321    4322    4323

Therapy engine

| Phase determination | Waveform determination | Ventilation determination |
| Flow limitation determination | Apnea / hypopnea determination | Snore determination |
| Airway patency determination | Target ventilation determination | Therapy parameter determination |

4324    4325    4326

4327    4328    4329

4340

Therapy control

Fault condition detection

4330

# FIG. 4D

4500

4520

Y ← Apnea / hypopnea index > threshold for a time?

N

4530    4560

Obstruction Index > threshold ? — N → Decrease treatment pressure

Y

4540

Airway patent? — N → Increase treatment pressure

Y

4550

**FIG. 4E**

**FIG. 5A**

**FIG. 5B**

**FIG. 5C**

**FIG. 5D**

**FIG. 5E**

FIG. 5G

FIG. 5H

FIG. 5I

**FIG. 5J**

**FIG. 5K**

**FIG. 5M**

**FIG. 6A**

**FIG. 6B**

**FIG. 6C**

**FIG. 6D**

5332

5700

5334

5700

5332

**FIG. 7A**

5334

5700

5332

5332

**FIG. 7B**

5310

5700

5600

5602

5000

5314

**FIG. 7C**

**FIG. 8A**

**FIG. 8B**

5602

5600

5802

5606

5604

5314

**FIG. 8C**

5808

5800

5806

5314

5808

5808

**FIG. 8D**

**FIG. 9**

INVERTED

RETURNED TO
WORKING
ORIENTATION

**FIG. 10**

**FIG. 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4944310 A, Sullivan
- US 6532959 B, Berthon-Jones
- WO 1998004310 A
- WO 2006074513 A
- WO 2010135785 A
- US 4782832 A, Trimble
- WO 2004073778 A
- US 20090044808 A
- WO 2005063328 A
- WO 2006130903 A
- WO 2009052560 A
- US 20100000534
- WO 1998034665 A
- WO 2000078381 A
- US 6581594 B
- US 20090050156 A
- US 20090044808
- US 2011100363 A1
- US 20191143070 A1
- US 2019209802 A1
- US 7866944 B
- US 8638014 B
- US 8636479 B
- WO 2013020167 A
- US 8733349 B
- WO 2012171072 A
- WO 2018094452 A

**Non-patent literature cited in the description**

- **JOHN B. WEST**. Respiratory Physiology. Lippincott Williams & Wilkins, 2012